(19) Europäisches Patentamt
European Patent Office
Office européen des brevets

(11) **EP 2 668 905 A1**

(12) **EUROPEAN PATENT APPLICATION**
published in accordance with Art. 153(4) EPC

(43) Date of publication:
**04.12.2013 Bulletin 2013/49**

(21) Application number: **12739623.2**

(22) Date of filing: **19.01.2012**

(51) Int Cl.:
***A61B 8/00*** (2006.01)

(86) International application number:
**PCT/JP2012/000314**

(87) International publication number:
**WO 2012/101989 (02.08.2012 Gazette 2012/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2011 JP 2011014431**

(71) Applicant: **Hitachi Medical Corporation**
**Chiyoda-ku**
**Tokyo 101-0021 (JP)**

(72) Inventor: **TSUJITA, Takehiro**
**Tokyo 101-0021 (JP)**

(74) Representative: **Strehl Schübel-Hopf & Partner**
**Maximilianstraße 54**
**D-80538 München (DE)**

(54) **ULTRASONIC DIAGNOSTIC DEVICE AND IMAGE PROCESSING METHOD**

(57)     Provided is a technology capable of obtaining at high speed a three-dimensional image having good image quality, without increasing the load on an ultrasonic diagnostic device. A plurality of filter processing having different effects are performed, prior to rendering, on tomographic volume data that has completed coordinate transformation, and volume data is obtained for each. A prescribed weight is added to each voxel for each of the obtained volume data, and a three-dimensional image is generated from the volume data obtained thereby. The filter processing having different effects may be different types of filter processing such as smoothing and sharpening, the same filter processing but having different strengths, or a combination of filter processing and no filter processing.

FIG. 2

CONTROL UNIT 0003

0014 THREE-DIMENSIONAL IMAGE PROCESSING UNIT

THREE-DIMENSIONAL COORDINATE CONVERSION UNIT 0013

0201 THREE-DIMENSIONAL SMOOTHING UNIT

0202 WEIGHTED ADDITION UNIT

0203 GRADIENT CALCULATING UNIT

0204 RENDERING UNIT

DISPLAY DATA GENERATING UNIT 0016

DISPLAY DEVICE 0009

EP 2 668 905 A1

## Description

### Technical Field

The present invention relates to an ultrasonic diagnostic device that builds an ultrasonic image of a diagnostic region in an object using ultrasound and displays the ultrasonic image, and more particularly, to an ultrasonic diagnostic device that can build three-dimensional ultrasonic images of a tomographic image, a blood flow image, and elasticity image data.

### Background Art

An ultrasonic diagnostic device transmits ultrasound to an object using an ultrasonic probe, receives a reflected echo signal from the object, and generates a tomographic image of the object using the reflected echo signal. In the ultrasonic diagnostic device, the ultrasonic probe is automatically or manually operated in a minor-axis direction, whereby three-dimensional data is obtained. The obtained three-dimensional data is projected on a two-dimensional projection surface by a method such as volume rendering to display a projected image (a three-dimensional image). For example, blood flow speed and blood flow strength in a blood flow image or information concerning distortion and hardness in an elasticity image are three-dimensionally acquired to generate and display a projected image.

At present, signal processing (image processing) from the reflected echo signal to the projected image generation is carried out in real time. A technique called real-time 3D or 4D for displaying a three-dimensional image as a moving image is generally adopted.

However, an ultrasonic image includes a large number of artifacts peculiar to ultrasound such as speckle noise, causing deterioration in image quality of a three-dimensional image. In order to reduce such noise and improve the image quality, three-dimensional filter processing such as smoothing having a relatively light processing load is performed. However, for example, the smoothing reduces the noise of the three-dimensional image and improves the image quality but, on the other hand, sometimes makes a boundary unclear. Since a data amount of three-dimensional data is enormous, it is difficult to perform, in real time, arbitrary image processing for improving the image quality.

As a technique for solving such a problem, for example, there is a method of determining, for each space, whether voxel data smoothed by a moving average method is amplified and used and improving image quality (see, for example, Patent Literature 1). In this method, it is determined according to a standard deviation of peripheral luminance whether the voxel data is used.

There is a method of improving image quality of a three-dimensional image by coupling a plurality of pieces of volume data when sufficient three-dimensional volume data is not obtained by one scanning (see, for example, Patent Literature 2).

Further, there is a technique for improving image quality of a three-dimensional image by combining two images, i.e., a volume rendering image in which a shape of an object can be grasped and a volume rendering image in which structure of the object can be grasped (see, for example, Patent Literature 3).

### Citation List

### Patent Literature

Patent Literature 1: JP-A-2007-229283
Patent Literature 2: JP-A-2009-61182
Patent Literature 3: JP-A-2006-130071

### Summary of Invention

### Technical Problem

When processing is excessive, even if an implementation region of smoothing is switched for each space, a harmful effect of the smoothing that an image becomes unclear cannot be removed. There is no choice but to select smoothing intensity out of smoothing intensities prepared in advance. There is no degree of freedom of the selection. Therefore, it is difficult to apply smoothing at optimum intensity.

The coupling processing disclosed in Patent Literature 2 is performed by averaged voxel values of the same region acquired at different times. Therefore, although a temporal smoothing effect is obtained, a spatial smoothing effect is not obtained.

When the combining processing disclosed in Patent Literature 3 is carried out, rendering processing needs to be carried out twice. Since a computational amount of the rendering processing is larger compared with three-dimensional filter processing such as smoothing, a load on a device is large. The rendering processing is not suitable for implementation in real time.

The present invention has been devised in view of the above and it is an object of the present invention to provide a technique that can acquire a three-dimensional image having satisfactory image quality at high speed without increasing a load on an ultrasonic diagnostic device.

Solution to Problem

The present invention applies, before rendering processing, a plurality of kinds of filter processing having different effects to tomographic volume data and obtains volume data in the respective kinds of filter processing. The present invention generates a three-dimensional image from volume data obtained by giving weight set in advance to the obtained respective volume data and adding up the volume data for each voxel.

The kinds of filter processing having the different effects may be kinds of processing having different kinds of filters such as smoothing and sharpening or may be the same kinds of filter processing having different intensities. The kinds of filter processing may be filter processing to be performed and filter processing not to be performed.

Specifically, there is provided an ultrasonic diagnostic device including: an ultrasonic probe; a transmitting unit configured to transmit a driving signal to the ultrasonic probe; a three-dimensional tomographic data generating unit configured to generate, using an ultrasonic signal from a test target measured by the ultrasonic probe, tomographic volume data of the test target; a three-dimensional image processing unit configured to generate a three-dimensional image from the tomographic volume data; and a display unit configured to display the three-dimensional image generated by the three-dimensional image processing unit. The three-dimensional image processing unit includes: a smoothing unit configured to apply spatial smoothing to the tomographic volume data and generate smoothed volume data; a weighted addition unit configured to multiply, when the smoothed volume data is generated, the tomographic volume data and the smoothed volume data with a weight coefficient and add up the volume data to generate combined volume data; and a rendering unit configured to apply, when the combined volume data is generated, rendering processing to the combined volume data and generate the three-dimensional image.

According to another aspect, there is provided an ultrasonic diagnostic device including: an ultrasonic probe; a transmitting unit configured to transmit a driving signal to the ultrasonic probe; a three-dimensional tomographic data generating unit configured to generate, using an ultrasonic signal from a test target measured by the ultrasonic probe, tomographic volume data of the test target; a three-dimensional image processing unit configured to generate a three-dimensional image from the tomographic volume data; and a display unit configured to display the three-dimensional image generated by the three-dimensional image processing unit. The three-dimensional image processing unit includes: a filter processing unit configured to apply each of a plurality of kinds of three-dimensional filter processing to the tomographic volume data and generate processed volume data in the respective kinds of three-dimensional filter processing; a weighted addition unit configured to multiply, when a plurality of pieces of the processed volume data are generated, the plurality of pieces of processed volume data with a weight coefficient and add up the plurality of pieces of processed volume data to generate combined volume data; and a rendering unit configured to apply, when the combined volume data is generated, rendering processing to the combined volume data and generate the three-dimensional image.

According to still another aspect, the present invention provides there is provided an ultrasonic diagnostic device including: an ultrasonic probe; a transmitting unit configured to transmit a driving signal to the ultrasonic probe; a three-dimensional tomographic data generating unit configured to generate, using an ultrasonic signal from a test target measured by the ultrasonic probe, tomographic volume data of the test target; a three-dimensional image processing unit configured to generate a three-dimensional image from the tomographic volume data; and a display unit configured to display the three-dimensional image generated by the three-dimensional image processing unit. The three-dimensional image processing unit includes: a filter processing unit configured to apply each of a plurality of kinds of three-dimensional filter processing to the tomographic volume data and generate processed volume data in the respective kinds of three-dimensional filter processing; and a rendering unit configured to apply, when the processed volume data is generated, rendering processing to a plurality of pieces of the generated processed volume data while multiplying the processed volume data with a weight coefficient and adding up the processed volume data for each constituent voxel to generate the three-dimensional image.

Further, there is provided an image processing method in an ultrasonic diagnostic device for transmitting ultrasound to a test target, applying image processing to tomographic volume data of the test target generated using a received ultrasonic signal, and generating a three-dimensional image. The image processing method includes: a smoothing step for applying spatial smoothing to the tomographic volume data and generating smoothed volume data; a weighted addition step for multiplying the tomographic volume data and the smoothed volume data with a weight coefficient and adding up the volume data to generate combined volume data; and a rendering step for applying rendering processing to the combined volume data and generating the three-dimensional image.

Further, there is provided a program for an ultrasonic diagnostic device for causing a computer to function as: a three-dimensional tomographic data generating unit configured to transmit ultrasound to a test target and generate tomographic volume data of the test target using a received ultrasonic signal; a smoothing unit configured to apply spatial smoothing to the tomographic volume data and generate smoothed volume data; a weighted addition unit configured to multiply the tomographic volume data and the smoothed volume data with a weight coefficient and add up the volume data to generate combined volume data; and a rendering unit configured to apply rendering processing to the combined volume data and generate the three-dimensional image.

Advantageous Effects of Invention

According to the present invention, it is possible to acquire a three-dimensional image having satisfactory image quality at high speed without increasing a load on an ultrasonic diagnostic device.

Brief Description of Drawings

[Figure 1] Figure 1 is a block diagram of an ultrasonic diagnostic device in a first embodiment.
[Figure 2] Figure 2 is a functional block diagram of a three-dimensional image processing unit in the first embodiment.
[Figure 3] Figure 3 is a flowchart of three-dimensional image processing in the first embodiment.
[Figure 4] Figures 4(a) to 4(c) are diagrams for explaining volume data generated by the three-dimensional image processing in the first embodiment.
[Figure 5] Figures 5(a) to 5(c) are diagrams for explaining a three-dimensional image generated in the first embodiment.
[Figure 6] Figures 6(a) to 6(c) are diagrams for explaining a weight coefficient setting interface in the first embodiment.
[Figure 7] Figures 7(a) to 7(c) are diagrams for explaining a smoothing coefficient setting interface in the first embodiment.
[Figure 8] Figure 8 is a functional block diagram of a three-dimensional image processing unit in a second embodiment.
[Figure 9] Figure 9 is a flowchart of three-dimensional image processing in the second embodiment.
[Figure 10] Figure 10 is a functional block diagram of a three-dimensional image processing unit in a third embodiment.
[Figure 11] Figure 11 is a flowchart of three-dimensional image processing in the third embodiment.
[Figure 12] Figures 12(a) to 12(c) are diagrams for explaining a smoothing method and sharpening method setting interface in the third embodiment.
[Figure 13] Figure 13 is a functional block diagram of a three-dimensional image processing unit in a fourth embodiment.
[Figure 14] Figure 14 is a flowchart of three-dimensional image processing in the fourth embodiment.
[Figure 15] Figure 15 is a diagram for explaining volume data generated in the fourth embodiment.

Description of Embodiments

«First Embodiment»

An embodiment of the present invention is explained below on the basis of the drawings. In the following explanation, in all the figures for explaining the embodiment of the present invention, components having the same functions are denoted by the same reference numerals and signs and repeated explanation of the components is omitted. In this embodiment, a three-dimensional image is generated from combined volume data generated by giving weight set in advance to tomographic volume data after three-dimensional image conversion and smoothed data of the tomographic volume and adding up the data. An ultrasonic diagnostic device includes an ultrasonic probe, a transmitting unit configured to transmit a driving signal to the ultrasonic probe, a three-dimensional tomographic data generating unit configured to generate, using an ultrasonic signal from a test target measured by the ultrasonic probe, tomographic volume data of the test target, a three-dimensional image processing unit configured to generate a three-dimensional image from the tomographic volume data, and a display unit configured to display the three-dimensional image generated by the three-dimensional image processing unit.

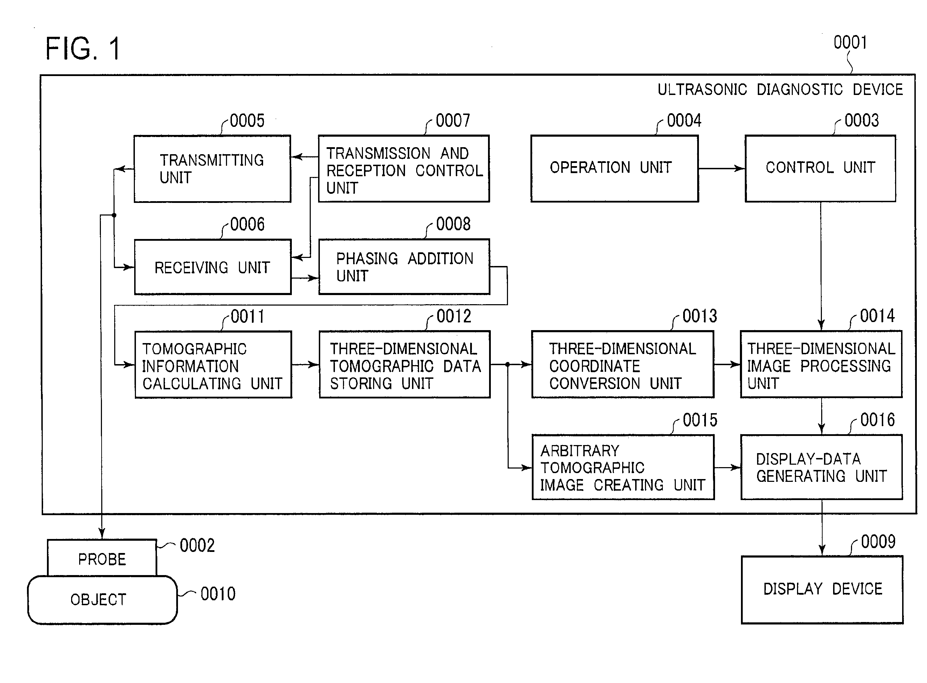

First, an ultrasonic diagnostic device 0001 in this embodiment is explained using Figure 1. As shown in Figure 1, the ultrasonic diagnostic device 0001 includes an ultrasonic probe 0002 used in contact with an object 0010, a transmitting unit 0005 configured to repeatedly transmit ultrasound to the object 0010 at a fixed time interval via the ultrasonic probe 0002, a receiving unit 0006 configured to receive a reflected echo signal reflected from the object 0010, a transmission and reception control unit 0007 configured to control operations of the transmitting unit 0005 and the receiving unit 0006, and a phasing addition unit 0008 configured to subject the reflected echo signal received by the receiving unit 0006 to phasing addition.

The ultrasonic probe 0002 includes a plurality of oscillators. The ultrasonic probe 0002 transmits ultrasound to the object 0010 via the oscillators and receives a reflected echo signal. The plurality of oscillators are arrayed in a rectangular shape or a fan shape. The ultrasonic probe 0002 mechanically swings the oscillators in a direction (a minor-axis direction) orthogonal to a direction of the array to thereby three-dimensionally transmit and receive ultrasound. Note that, as the ultrasonic probe 0002, an ultrasonic probe may be used in which a plurality of oscillators are two-dimensionally arrayed and that electronically controls transmission and reception of ultrasound to three-dimensionally transmit and receive the ultrasound.

The transmitting unit 0005 transmits a driving signal to the ultrasonic probe 0002. The transmitting unit 0005 generates a wave transmission pulse for driving the oscillators of the ultrasonic probe 0002 to generate ultrasound. When generating the wave transmission pulse, the transmitting unit 0005 controls a phase of a wave transmission signal passed to the respective oscillators of the ultrasonic probe 0002 and sets a convergent point of transmitted ultrasound to certain depth. The receiving unit 0006 amplifies, with a predetermined gain, reflected echo signals received by the respective oscillators of the ultrasonic probe 0002 and generates RF signals, i.e., reception signals. The transmission and reception control unit 0007 controls operations of the transmitting unit 0005 and the receiving unit 0006. The phasing addition unit 0008 adds up, after aligning phases of the RF signals amplified by the receiving unit 0006, the RF signals to thereby form an ultrasonic beam converging at one point or a plurality of convergent points and generate RF signal frame data (equivalent to RAW data).

The three-dimensional tomographic data generating unit generates, using an ultrasonic signal from a test target measured by the ultrasonic probe 0002, tomographic volume data of the test target. The ultrasonic diagnostic device 0001 includes a tomographic information calculating unit 0011, a three-dimensional tomographic data storing unit 0012, and a three-dimensional coordinate conversion unit 0013 as components of the three-dimensional tomographic data generating unit configured to generate tomographic volume data, which is three-dimensional data of a diagnostic region, from the RF signal frame data. The ultrasonic diagnostic device 0001 includes a three-dimensional image processing unit 0014 and a display data generating unit 0016 as components configured to generate a three-dimensional image (a two-dimensional projected image of three-dimensional volume data) of a diagnostic region having amplitude of received ultrasound as luminance, from the tomographic volume data. Further, the ultrasonic diagnostic device 0001 may include an arbitrary tomographic image creating unit 0015 as a component configured to create a two-dimensional image of an arbitrary tomographic profile. Details of the respective units are explained below.

The ultrasonic diagnostic device 0001 includes an operation unit 0004 and a display device (a display unit) 0009 as user interfaces. Further, the ultrasonic diagnostic device 0001 includes a control unit 0003 configured to control operation of the entire ultrasonic diagnostic device 0001.

The tomographic information calculating unit 0011 applies signal processing such as gain correction, log compression, detection, edge enhancement, and smoothing to the RF signal frame data generated by the phasing addition unit 0008 and forms secondary tomographic data. In that case, the control unit 0003 controls the tomographic information calculating unit 0011 according to a setting condition received from an operator via the operation unit 0004.

The three-dimensional tomographic data storing unit 0012 stores the two-dimensional tomographic data formed by the tomographic information calculating unit 0011 while associating the two-dimensional tomographic data with an acquisition position thereof. The acquisition position is a transmitting and receiving direction. For example, when a coordinate system including a $\theta$ direction and a $\phi$ direction orthogonal to the $\theta$ direction is used, if two-dimensional tomographic data is formed by a measurement result obtained by performing transmission and reception in the $\theta$ direction, the three-dimensional tomographic data storing unit 0012 stores, for each position in the $\phi$ direction orthogonal to the $\theta$ direction, a plurality of pieces of two-dimensional tomographic data using the position as an index.

The three-dimensional coordinate conversion unit 0013 applies three-dimensional coordinate conversion to the plurality of pieces of two-dimensional tomographic data stored in the three-dimensional tomographic data storing unit 0012 and generates tomographic volume data.

The three-dimensional image processing unit 0014 applies three-dimensional image processing to the tomographic volume data after the coordinate conversion in the three-dimensional coordinate conversion unit 0013 and generates a three-dimensional image. The three-dimensional image processing unit 0014 executes the three-dimensional image processing every time tomographic volume data is generated and generates a three-dimensional image. Details of the three-dimensional image processing in this embodiment are explained below. A color scale, brightness of which increase mainly according to luminance, such as a gray scale changing from black to white according to luminance or a sepia color having a reddish tinge is given to the generated three-dimensional image.

The arbitrary tomographic image creating unit 0015 creates, using the two-dimensional tomographic data stored in the three-dimensional tomographic data storing unit 0012, a two-dimensional image of a tomographic profile designated by the operator. Note that the operator sets a conversion coefficient via the operation unit 0004 to thereby designate a displayed tomographic profile.

The display data generating unit 0016 generates display data for causing the display device 0009 to display the three-dimensional image generated in the three-dimensional image processing unit 0014. The display data generating unit 0016 in this embodiment generates display data every time a three-dimensional image is generated and updates display data of the display device 0009. Note that, when the ultrasonic diagnostic device 0001 includes the arbitrary tomographic image creating unit 0015, the display data generating unit 0016 generates, as display data, display data for displaying the three-dimensional image together with the two-dimensional image generated in the arbitrary tomographic image creating unit 0015. For example, the display data generating unit 0016 displays the two-dimensional image and the three-dimensional image in parallel to each other.

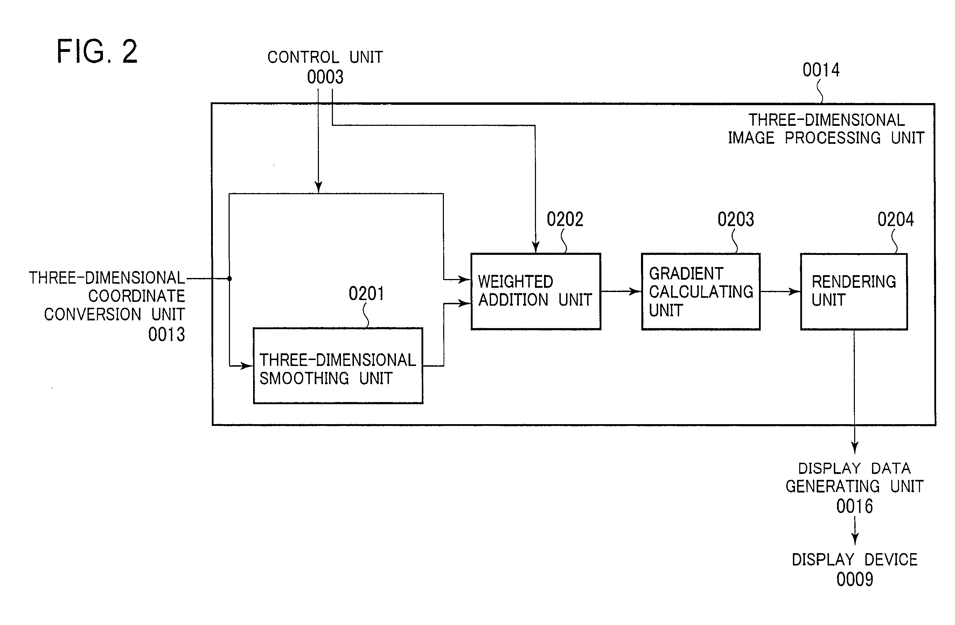

Next, the three-dimensional image processing unit 0014 characteristic in this embodiment is explained using Figure 2. Figure 2 is a functional block diagram of the three-dimensional image processing unit 0014 in this embodiment.

As shown in the figure, the three-dimensional image processing unit 0014 in this embodiment performs three-dimensional image processing for combining, at an arbitrary ratio, tomographic volume data itself generated by the three-dimensional coordinate conversion unit 0013 and data, which is obtained by applying smoothing to the tomographic volume data, and generating a three-dimensional image. The three-dimensional image processing unit 0014 includes a three-dimensional smoothing unit 0201, a weighted addition unit 0202, and a gradient calculating unit 0203 for realizing the three-dimensional image processing. Hereinafter, in this embodiment, the tomographic volume data subjected to the smoothing is referred to as smoothed volume data (smooth volume data) and the tomographic volume data itself not subjected to the smoothing is referred to as texture volume data (original volume data).

The three-dimensional smoothing unit (the filter processing unit) 0201 applies spatial smoothing (three-dimensional smoothing) to the tomographic volume data after the coordinate conversion in the three-dimensional coordinate conversion unit 0013 and generates smoothed volume data. The three-dimensional smoothing unit (the filter processing unit) 0201 may apply each of a plurality of kinds of three-dimensional filter processing to the tomographic volume data and generate processed volume data in the respective kinds of three-dimensional filter processing. In this embodiment, as the smoothing, for example, averaging filter processing for averaging the tomographic volume data using voxel values around the tomographic volume data is used. Intensity of smoothing of the averaging filter processing depends on the number of averaged voxels. The number of averaged voxels is set by the operator as a smoothing coefficient.

The weighted addition unit 0202 performs weighted addition processing for subjecting a plurality of pieces of volume data generated from the same tomographic volume data to weighted addition using a set weight coefficient and generating volume data. The weighted addition unit 0202 performs the weighted addition for each voxel of volume data. The weighted addition unit 0202 multiplies, every time smoothed volume data is generated, the tomographic volume data and the smoothed volume data with the weight coefficient and adds up the volume data to generate combined volume data. The weighted addition unit 0202 multiplies, every time a plurality of pieces of processed volume data are generated, the plurality of pieces of processed volume data with the weight coefficient and adds up the volume data to generate combined volume data. Hereinafter, in this embodiment, volume data obtained by the weighed addition processing is referred to as combined volume data. In this embodiment, the plurality of pieces of volume data generated from the same tomographic volume data are two volume data, i.e., texture volume data, which is the tomographic volume data itself, and smoothed volume data generated from the tomographic volume data by the three-dimensional smoothing unit 0201.

As the weight coefficient, the control unit 0003 notifies the weighted addition unit 0202 of a value set by the operator via the operation unit 0004. That is, the operation unit (the interface) 0004 receives an input of the weight coefficient. The weight coefficient is set as, for example, a value between 0 and 1. The addition is performed according to the following Equation (1) for each voxel corresponding to both the volume data. Here, the weight coefficient is represented as W, a luminance value of one volume data of a predetermined voxel v is represented as $V1v$, a luminance value of the other volume data is represented as $V2v$, and a luminance value of the combined volume data is represented as $Cv$.

$$Cv = V1v \cdot W + V2v \cdot (1-W) \qquad (1)$$

The gradient calculating unit 0203 generates gradient information of the combined volume data. The gradient information is a gradient (a gradient value) of a luminance value for each voxel calculated from luminance values of respective voxels forming the combined volume data and luminance values of voxels around the respective voxels.

A rendering unit 0204 applies volume rendering processing to the combined volume data and generates a three-dimensional image. The rendering unit 0204 may apply, every time combined volume data is generated, the rendering processing to the combined volume data and generate the three-dimensional image. The rendering unit 0204 may apply, every time processed volume data is generated, the rendering processing to a plurality of pieces of the generated processed volume data while multiplying the processed volume data with the weight coefficient and adding up the processed volume data for each constituent voxel to generate the three-dimensional image. Note that the generated three-dimensional image is displayed on the display device 0009 via the display data generating unit 0016.

The rendering unit 0204 applies the volume rendering processing to the combined volume data using the following Formula (2) and Formula (3) to thereby form a three-dimensional image of a diagnostic region of the object. The control unit 0003 receives a projecting direction (a line of sight direction) from the operator via the operation unit 0004.

$$C_{out} = C_{out-1} + (1 - A_{out-1}) \cdot A_i \cdot C_i \cdot S_i \qquad (2)$$

$$A_{out} = A_{out-1} + (1 - A_{out-1}) \cdot A_i \quad (3)$$

In the above equations, $C_i$ represents a luminance value of an ith voxel present on a line of sight when the combined volume data is viewed from a certain point on a created two-dimensional projection surface. When data of N voxels are arranged on the line of sight, a value $C_{out}$ obtained by integrating i = 0 to N is a final output pixel value. $C_{out-1}$ indicates an integrated value up to an i-1th voxel.

In the above equations, $A_i$ represents opacity of the ith voxel present on the line of sight. The opacity $A_i$ takes a value from 0 to 1.0. A relation between voxel values and opacity is set in advance as an opacity table in which a relation between luminance values and opacity is set. The rendering unit 0204 obtains opacity from a luminance value of a voxel referring to the opacity table.

$S_i$ indicates a gradient value of the ith voxel present on the line of sight. For example, when a normal of a surface specified by a gradient of the luminance value of the ith voxel coincides with the line of sight, for example, 1.0 is given to $S_i$. When the normal is orthogonal to the line of sight, for example, 0 is given to $S_i$. As a gradient with respect to the line of sight is larger, a gradient value decreases and a calculated output pixel value is a smaller value. Note that, in this embodiment, a configuration not including the gradient calculating unit 0203 is also possible. In this case, a fixed value, for example, 1.0 is always given to $S_i$.

Note that initial values of both $C_{out}$ and $A_{out}$ are 0.

As indicated by Equation (3), $A_{out}$ is integrated every time the volume rendering processing passes through a voxel and converges to 1.0. Therefore, as indicated by Equation (2), the integrated value $A_{out-1}$ of the opacity up to the i-1th voxel is $\equiv$1.0, the ith voxel value $C_i$ is not reflected on an output image. According to such volume rendering processing, it is possible to grasp a voxel having high opacity as a surface and stereoscopically display three-dimensional tomographic image data.

The finally obtained $C_{out}$ is subjected to color scale conversion, converted into color information such as RGB, and output.

Note that the rendering unit 0204 may generate a three-dimensional image for each element of the color information such as RGB according to the following Equations (4) to (6):

$$CR_{out} = CR_{out-1} + (1 - A_{out-1}) \cdot A_i \cdot CR_i \cdot S_i \qquad (4)$$

$$CG_{out} = CG_{out-1} + (1 - A_{out-1}) \cdot A_i \cdot CG_i \cdot S_i \qquad (5)$$

$$CB_{out} = CB_{out-1} + (1 - A_{out-1}) \cdot A_i \cdot CB_i \cdot S_i \qquad (6)$$

Here, $CR_i$ represents a red component of a luminance value of the ith voxel present on the line of sight when the combined volume data is viewed from a certain point on a created two-dimensional projection surface. $CG_i$ and $CB_i$ respectively represent a green component and a blue component.

Note that a method in which the rendering unit 0204 in this embodiment generates a three-dimensional image from the combined volume data is not limited to this. For example, the method may be a maximum value projection method (Maximum intensity projection) for making a structure on an inside rather than on a surface transparently visible and displaying only a high-luminance structure in a region of interest, a minimum value projection method (Minimum intensity projection) for rendering only a low-luminance structure, or a method (Ray summation) for displaying a cumulative image of voxel values in the line of sight direction.

The rendering unit 0204 may be configured to discriminate, in a process of the rendering processing, luminance values of voxels of respective processing targets according to a threshold and select whether data of the voxels is enabled or disabled. The operator sets the threshold via the operation unit 0004. The control unit 0003 notifies the rendering unit 0204 of the threshold.

Note that the ultrasonic diagnostic device 0001 in this embodiment includes a CPU, a memory, and a storage device. Further, the ultrasonic diagnostic device 0001 may include a GPU (Graphics Processing Unit). Functions of the control unit 0003 and the respective units (the tomographic information calculating unit 0011, the three-dimensional coordinate conversion unit 0013, the three-dimensional image processing unit 0014, the arbitrary tomographic image creating unit 0015, and the display data generating unit 0016) for generating tomographic volume data from the RF signal frame data and further generating a two-dimensional image and a three-dimensional image are realized by the CPU or the GPU loading a program stored in the storage device in advance to the memory and executing the program.

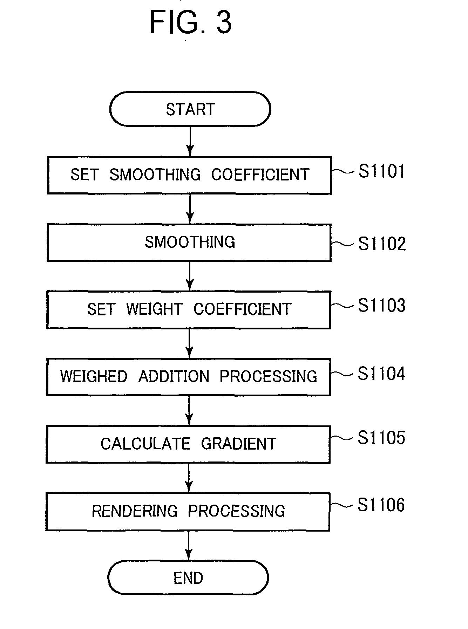

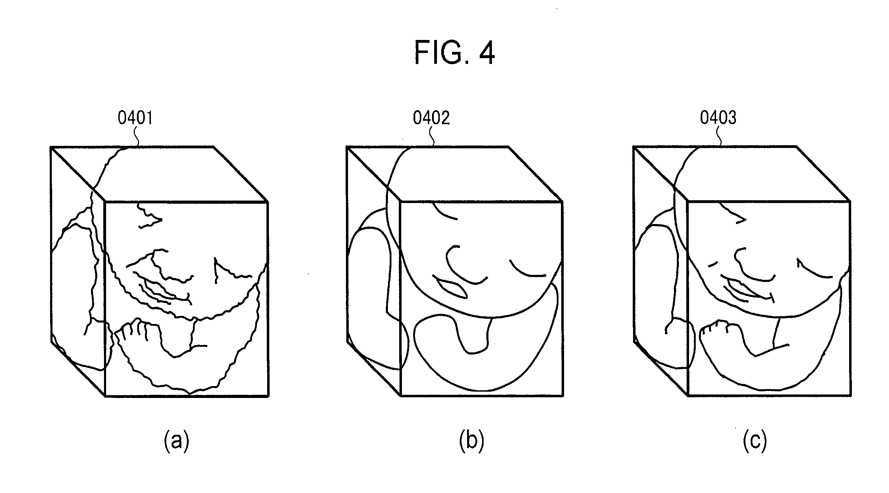

Next, a flow of three-dimensional image processing by the three-dimensional image processing unit 0014 in this embodiment and an image generated by the three-dimensional image processing are explained using Figure 3 and Figure 4.

Figure 3 is a processing flow of the three-dimensional image processing by the three-dimensional image processing unit 0014 in this embodiment. Figure 4 is a diagram for explaining volume data obtained by the three-dimensional image processing by the three-dimensional image processing unit 0014 in this embodiment. Figure 4(a) is tomographic volume data (texture volume data) 0401, which is output data of the three-dimensional coordinate conversion unit 0013.

The three-dimensional smoothing unit (the filter processing unit) 0201 sets a smoothing coefficient (step S1101). The smoothing coefficient is designated by the operator via the operation unit 0004 out of smoothing coefficients set in advance. The three-dimensional smoothing unit 0201 receives the designated smoothing coefficient via the control unit 0003 and sets the smoothing coefficient. Note that, in this embodiment, in order to execute averaging filter processing as the smoothing, the number of averaged voxels is set as the smoothing coefficient as explained above.

Next, the three-dimensional smoothing unit 0201 applies the smoothing to tomographic volume data, which is output data of the three-dimensional coordinate conversion unit 0013 and generates smoothed volume data (step S1102). Figure 4(b) is smoothed volume data 0402 after the smoothing by the three-dimensional smoothing unit 0201.

The weighted addition unit 0202 receives and sets the weight coefficient (step S1103).

The weight coefficient is set by the operator via the operation unit 0004. The weighted addition unit 0202 receives the weight coefficient via the control unit 0003 and sets the weight coefficient.

The weighted addition unit 0202 performs addition processing indicated by above Equation (1) for each voxel using the set weight coefficient (step S1104), adds up the smoothed volume data and the texture volume data, and generates combined volume data. Combined volume data 0403 generated here is shown in Figure 4(c).

For example, as shown in Figure 4(b), even if the smoothed volume data 0402 is excessively subjected to the smoothing, the smoothed volume data 0402 is subjected to the addition processing by the weighted addition unit 0202, whereby it is possible to obtain the smoothed combined volume data 0403 intermediate between the smoothed volume data 0402 and the texture volume data 0401. If the weigh coefficient is changed, it is possible to obtain combined volume data having desired smoothing intensity intermediate between the smoothed volume data 0402 and the texture volume data 0401.

The gradient calculating unit 0203 applies a luminance gradient calculation for a voxel to the combined volume data 0403 and generates gradient information (step S1105). Then, the rendering unit 0204 performs the rendering processing using the combined volume data 0403 and a gradient value for each voxel and generates a three-dimensional image (step S1106). The generated three-dimensional image is changed to display data by the display data generating unit 0016 and displayed on the display device 0009.

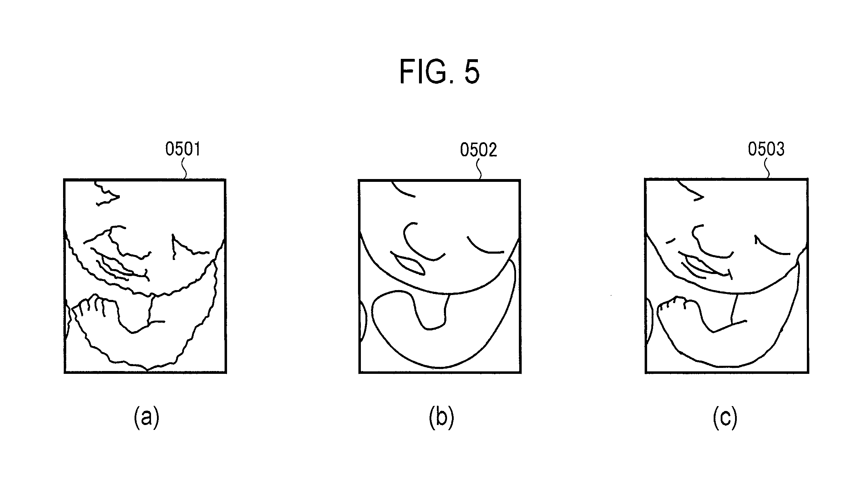

Next, an effect on a three-dimensional image given by a weight coefficient set by the operator is explained using Figure 5. In this embodiment, $V1v$ in Equation (1) is explained as a luminance value of the texture volume data 0401 and $V2v$ is explained as a luminance value of the smoothed volume data 0402.

A three-dimensional image 0501 shown in Figure 5(a) is a three-dimensional image generated when a weight coefficient W is set to 1.0. The three-dimensional image 0501 is generated from combined volume data obtained by weighting the texture volume data 0401 with 1.0 and weighting the smoothed volume data 0402 with 0.0 according to Equation (1).

When 1.0 is set as the weight coefficient W, as the combined volume data from which the three-dimensional image 0501 is generated, the texture volume data 0401 is directly used. Therefore, the three-dimensional image 0501 is generated from volume data not smoothed at all. Consequently, information such as wrinkles of fingers and a face of a fetus is retained but, on the other hand, unevenness remains on a boundary line of the image.

A three-dimensional image 0502 shown in Figure 5(b) is a three-dimensional image generated when the weight coefficient W is set to 0.0. The three-dimensional image 0502 is generated from combined volume data obtained by weighting the texture volume data 0401 with 0.0 and weighting the smoothed volume data 0402 with 1.0 according to Equation (1).

When 0.0 is set as the weight coefficient W, as the combined volume data from which the three-dimensional image 0502 is generated, the smoothed volume data 0402 is directly used. Therefore, the three-dimensional image 0502 is generated from volume data smoothed by a set smoothing coefficient. Consequently, the boundary line of the image is smooth without unevenness but, on the other hand, information such as the wrinkles of the fingers and the face of the fetus disappears.

A three-dimensional image 0503 shown in Figure 5(c) is a three-dimensional image generated when the moderate weight coefficient W, for example, 0.5 is set. The three-dimensional image 0503 is generated from combined volume data obtained by weighting the texture volume data 0401 with 0.5 and weighting the smoothed volume data 0402 with 0.5 according to Equation (1). As shown in the figure, in the obtained three-dimensional image 0503, unevenness on the boundary line moderately disappears and information such as the wrinkles of the fingers and the face of the fetus is retained.

As explained above, it is possible to adjust finally obtained image quality by adjusting the weight coefficient. In this embodiment, the operator adjusts the weight coefficient by changing the weight coefficient in real time while looking at an image displayed on the display device 0009.

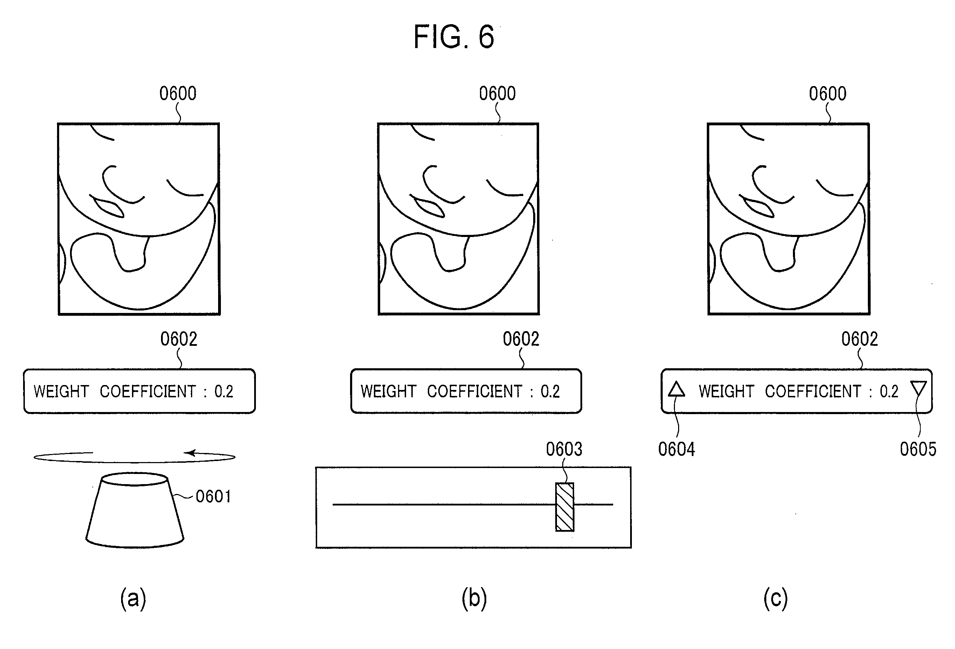

Next, an interface with which the operator sets and adjusts a weight coefficient is explained using Figure 6.

Figure 6(a) is an example in which the interface for setting and adjusting a weight coefficient is configured by a changing dial 0601, a coefficient display region 0602, and a three-dimensional image display region 0600. The operation unit 0004 includes the changing dial 0601. The operator changes the weight coefficient between 0.0 and 0.1 by operating the changing dial 0601.

The display device 0009 includes the coefficient display region 0602 and the three-dimensional image display region 0600. Note that the three-dimensional image display region 0600 is a region where a three-dimensional image generated by the three-dimensional image processing unit 0014 is displayed.

The control unit 0003 displays, every time the operator sets or changes a weight coefficient via the changing dial 0601, the weight coefficient in the coefficient display region 0602. The control unit 0003 notifies, every time the operator sets or changes a weight coefficient via the changing dial 0601, the three-dimensional image processing unit 0014 of the weight coefficient. The three-dimensional image processing unit 0014 receives the notification and repeats the processing from step S1103 of the flow of the three-dimensional image processing. The generated three-dimensional image is displayed in the three-dimensional image display region 0600. The operator sets an optimum weight coefficient while checking the three-dimensional image displayed in the three-dimensional image display region 0600.

Figure 6(b) is an example in which the interface for setting and adjusting a weight coefficient is configured by a touch panel on the display device 0009, the coefficient display region 0602, and the three-dimensional image display region 0600. The display device 0009 includes a slide bar 0603 for receiving an instruction for increasing and reducing a value of a coefficient. The operator changes a weight coefficient between 0.0 and 0.1 by operating the slide bar 0603 using a finger or an input device such as a mouse, a trackball, or a keyboard included in the operation unit 0004. Processing performed by the control unit 0003 when the weight coefficient is set or changed by the operator is the same as the processing in the example shown in Figure 6(a).

Figure 6(c) is an example in which the interface for setting and adjusting a weight coefficient is configured by the touch panel on the display device 0009, the coefficient display region 0602, and the three-dimensional image display region 0600. The display device 0009 includes an increase instruction region 0604 for receiving an instruction for increasing a value of a coefficient and a reduction instruction region 0605 for receiving an instruction for reducing the value of the coefficient. The operator changes a weight coefficient between 0.0 and 0.1 via the increase instruction region 0604 or the reduction instruction region 0605. The operator performs an instruction using a finger or the input device such as the mouse or the keyboard included in the operation unit 0004. Processing performed by the control unit 0003 when the weight coefficient is set or changed by the operator is the same as the processing in the example shown in Figure 6(a).

Note that, in the example shown in Figure 6(c), the increase instruction region 0604 and the reduction instruction region 0605 are included in the coefficient display region 0602. However, the increase instruction region 0604 and the reduction instruction region 0605 do not always have to be included in the coefficient display region 0602.

Note that the interface with which the operator sets and adjusts a weight coefficient is not limited to the above.

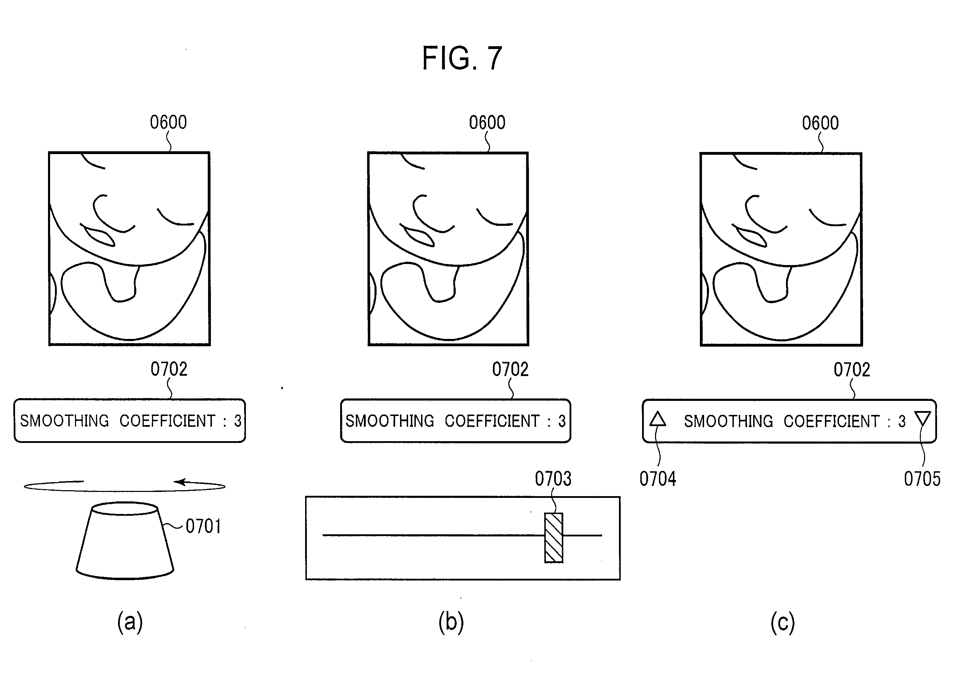

Note that the interface may be configured to enable the operator to change a smoothing coefficient as well in real time between smoothing coefficients prepared in advance while checking a three-dimensional image. In this case, like the weight coefficient, the operator sets an optimum smoothing coefficient by changing the smoothing coefficient in real time while looking at an image displayed on the display device 0009. In the following explanation, the interface with which the operator sets and changes a smoothing coefficient is explained using Figure 7.

Figure 7(a) is an example in which the interface for setting and changing a smoothing coefficient is configured by a changing dial 0701, a coefficient display region 0702, and the three-dimensional image display region 0600. The operation unit 0004 includes the changing dial 0701. The operator selects a smoothing coefficient out of smoothing coefficients set in advance using the changing dial 0701. For example, the smoothing coefficients set in advance are displayed on the display device 0009 as numbers. The control unit 0003 notifies the three-dimensional image processing unit 0014 of a  smoothing coefficient corresponding to a number selected by the operator.

The display device 0009 includes the coefficient display region 0702. The three-dimensional image display region 0600 is a region where a three-dimensional image generated by the three-dimensional image processing unit 0014 is displayed.

The control unit 0003 displays, every time the operator sets or changes a smoothing coefficient via the changing dial 0701, the smoothing coefficient in the coefficient display region 702. The control unit 0003 notifies, every time the operator sets or changes a smoothing coefficient via the changing dial 0701, the three-dimensional image processing unit 0014 of the smoothing coefficient. The three-dimensional image processing unit 0014 receives the notification and repeats the processing from step S1101 of the flow of the three-dimensional image processing. The generated three-dimensional image is displayed in the three-dimensional image display region 0600. The operator sets an optimum smoothing coefficient while checking the three-dimensional image displayed in the three-dimensional image display region 0600.

Figure 7(b) is an example in which the interface for setting and changing a smoothing coefficient is configured by the touch panel on the display device 0009, the coefficient display region 0702, and the three-dimensional image display region 0600. The display device 0009 includes a slide bar 0703 for receiving an instruction for increasing and reducing a value of a coefficient. The operator selects a smoothing coefficient out of the smoothing coefficients set in advance by operating the slide bar 0703 using a finger or the input device such as the mouse, the trackball, or the keyboard included in the operation unit 0004. Processing performed by the control unit 0003 when a smoothing coefficient is set or changed by the operator is the same as the processing in the example shown in Figure 7(a).

Figure 7(c) is an example in which the interface for setting and changing a smoothing coefficient is configured by the touch panel on the display device 0009, the coefficient display region 0702, and the three-dimensional image display region 0600. The display device 0009 includes an increase instruction region 0704 for receiving an instruction for increasing a value of a coefficient and a reduction instruction region 0705 for receiving an instruction for reducing the value of the coefficient. The operator selects a smoothing coefficient out of the smoothing coefficients set in  advance via the increase instruction region 0704 or the reduction instruction region 0705. The operator performs an instruction using a finger or the input device such as the mouse or the keyboard included in the operation unit 0004. Processing performed by the control unit 0003 when a smoothing coefficient is set or changed by the operator is the same as the processing in the example shown in Figure 7(a).

Note that, in the example shown in Figure 7(c), the increase instruction region 0704 and the reduction instruction region 0705 are included in the coefficient display region 0702. However, the increase instruction region 0704 and the reduction instruction region 0705 do not always need to be included in the coefficient display region 0702.

The interface with which the operator sets and changes a smoothing coefficient is not limited to the above.

The interface for setting and changing a smoothing coefficient may be used as the interface for setting and adjusting a weight coefficient as well. In this case, the interface includes a component configured to receive, from the operator, an instruction concerning which coefficient is changed. According to the instruction, the interface receives the instruction as a change of a coefficient corresponding to the instruction.

As explained above, according to this embodiment, in the three-dimensional image processing, combined volume data is generated by multiplying tomographic volume data and smoothed volume data, which is obtained by applying the smoothing to the tomographic volume data, with a weight coefficient and adding up the volume data. The rendering processing is applied to the generated combined volume data to generate a three-dimensional image.

In this embodiment, it is possible to change intensity of smoothing of combined volume data by changing a weight coefficient used during addition. Therefore, the operator can arbitrarily change, by arbitrarily setting a weight coefficient, information concerning unevenness that the operator desires to suppress and fine form information that the operator desires to retain. Since the weight coefficient used during addition can be arbitrarily changed in real time, it is possible to freely adjust smoothing intensity of the combined volume data in real time. Therefore, it is possible to easily obtain a three-dimensional image subjected to desired smoothing and obtain an optimum image according to necessity.

When a plurality of images after the rendering processing are combined, for example, when an integrated value of opacity is 1, a voxel value before the integrated value is not reflected on an output image. Therefore, even if the voxel value is subjected to weighted addition, the voxel value is not reflected on an image after the addition either. On the other hand, according to this embodiment, intensity of smoothing is adjusted in a state of volume data before rendering. Therefore, it is possible to obtain combined volume data having the adjusted intensity of the smoothing without losing information concerning respective voxels. Since a three-dimensional image is generated from the combined volume data, it is possible to obtain, from acquired all volume data, a three-dimensional image on which more information is reflected.

Texture data and smooth data to be subjected to weighted addition in order to adjust smoothing intensity are generated from the same tomographic volume data. Therefore, deterioration in image quality due to a temporal and/or spatial change does not occur.

Further, in the three-dimensional image processing for performing the smoothing, a component added anew is only the weighted addition high in speed and having little load on the device. The rendering processing having a large load on the device is performed only once as in the past. As explained above, a processing amount of the rendering processing is enormous. The rendering processing takes long time and has a large load on the device. Therefore, it is possible to obtain a three-dimensional image having desired image quality at high speed without increasing a load on the device.

Note that, in this embodiment, the averaging filter is used as the spatial smoothing in the three-dimensional smoothing unit 0201. However, the spatial smoothing is not limited to this. For example, a low-pass filter, a median filter, or the like may be three-dimensionally used. Expansion processing by dilation may be three-dimensionally performed.

«Second Embodiment»

Next, a second embodiment to which the present invention is applied is explained. In the first embodiment, the three-dimensional image processing unit 0014 combines, using a predetermined weight coefficient, tomographic volume data itself, which is an output of the three-dimensional coordinate conversion unit 0013, and volume data obtained by applying the smoothing to the tomographic volume data and generates combined volume data. On the other hand, in this embodiment, a three-dimensional image processing unit adds up, using a predetermined weight coefficient, two volume data respectively obtained by applying different kinds of smoothing to an output of the same three-dimensional coordinate conversion unit and generates combined volume data.

The ultrasonic diagnostic device 0001 in this embodiment basically has a configuration same as the configuration of the ultrasonic diagnostic device 0001 in the first embodiment. However, since three-dimensional image processing is different as explained above, the ultrasonic diagnostic device 0001 includes a three-dimensional image processing unit 0014a instead of the three-dimensional image processing unit 0014 in the first embodiment. In this embodiment, the three-dimensional image processing unit 0014a is mainly explained below.

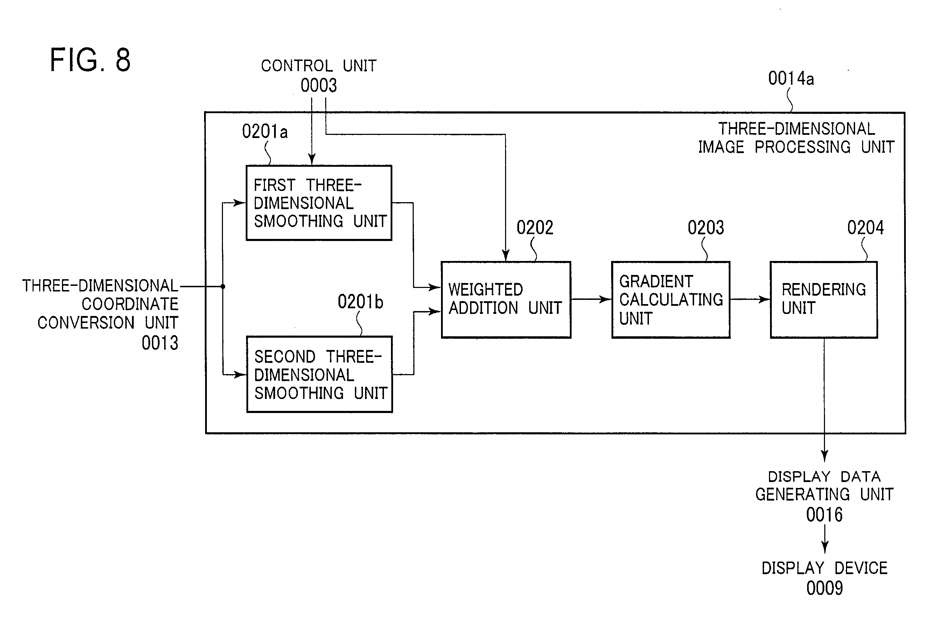

Figure 8 is a functional block diagram of the three-dimensional image processing unit 0014a in this embodiment. As shown in the figure, the three-dimensional image processing unit 0014a in this embodiment includes two three-dimensional smoothing units (filter processing units) 0201 (0201a and 0201b), the weighted addition unit 0202, the gradient calculating unit 0203, and the rendering unit 0204.

Each of functions of the two three-dimensional smoothing units 0201 is basically the same as the function of the three-dimensional smoothing unit (the filter processing unit) 0201 in the first embodiment. That is, the three-dimensional smoothing units 0201 apply spatial smoothing to tomographic volume data using a set smoothing coefficient. However, smoothing coefficients used in the two three-dimensional smoothing units (the filter processing units) 0201a and 0201b are different from each other. The three-dimensional smoothing units (the filter processing units) 0201 apply each of a plurality of kinds of three-dimensional filter processing to the tomographic volume data and respectively generate processed volume data.

Hereinafter, for explanation, a side where a smoothing coefficient with a weak smoothing effect (a first smoothing coefficient) is used is referred to as first three-dimensional smoothing unit 0201a and a side where a smoothing coefficient with a smoothing effect stronger than the smoothing effect of the first smoothing coefficient is used is referred to as second three-dimensional smoothing unit 0201b. Hereinafter, in this embodiment, volume data generated by the first three-dimensional smoothing unit 0201a is referred to as texture volume data (weakly smoothed volume data) and volume data generated by the second three-dimensional smoothing unit 0201b is referred to as smoothed volume data (intensely smoothed volume data).

Like the smoothing coefficient in the first embodiment, the first smoothing coefficient and the second smoothing coefficient are designated by an operator via the operation unit 0004 out of smoothing coefficients set in advance. The first three-dimensional smoothing unit 0201a and the second three-dimensional smoothing unit 0201b respectively receive the designated first smoothing coefficient and the designated second smoothing coefficient via the control unit 0003 and set the first smoothing coefficient and the second smoothing coefficient. As in the first embodiment, the number of averaged voxels in a smoothing filter is set in the first smoothing coefficient and the second smoothing coefficient.

A function of the weighted addition unit 0202 is basically the same as the function in the first embodiment.

However, in this embodiment, a plurality of pieces of volume data generated from the same tomographic volume data to be subjected to weighted addition are two volume data, i.e. texture volume data subjected to weak smoothing and smoothed volume data subjected to intense smoothing.

Functions of the other components (the gradient calculating unit 0203 and the rendering unit 0204) are basically the same as the functions in the first embodiment. As in the first embodiment, a configuration not including the gradient calculating unit 0203 is also possible.

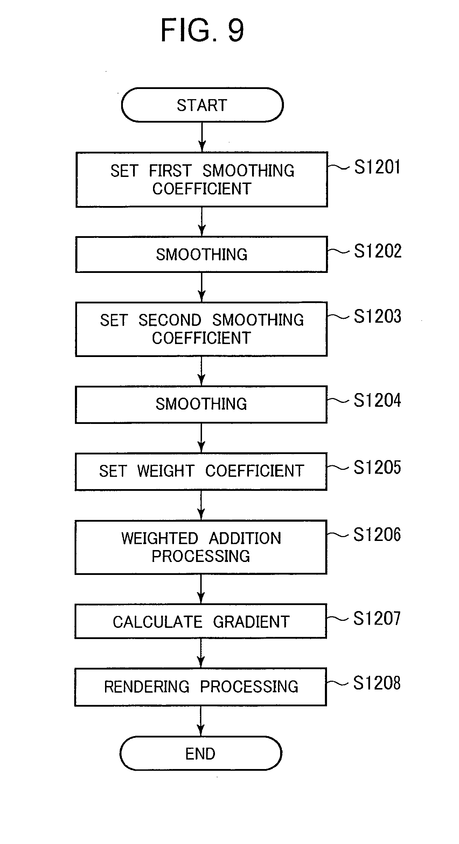

Next, a procedure of image processing by the three-dimensional image processing unit 0014a in this embodiment is explained using Figure 9. Figure 9 is a processing flow of image processing by the three-dimensional image processing unit 0014a in this embodiment.

Volume data to be generated is explained using Figure 4. Figure 4 is used for the explanation of the three-dimensional image processing unit 0014 in the first embodiment. However, Figure 4 is a figure for explaining an overview of an effect and does not indicate that the three-dimensional image processing unit 0014 in the first embodiment and the three-dimensional image processing unit 0014a in the second embodiment produce the same result.

The three-dimensional smoothing unit 0201a sets a first smoothing coefficient (step S1201), applies smoothing to tomographic volume data, which is output data of the three-dimensional coordinate conversion unit 0013, and generates the texture volume data 0401 shown in Figure 4(a) (step S1202).

Next, the three-dimensional smoothing unit 0201b sets a second smoothing coefficient (step S1203), applies smoothing to tomographic volume data, which is output data of the three-dimensional coordinate conversion unit 0013, and generates the smoothed volume data 0402 shown in Figure 4(b) (step S1204).

Note that processing order of the first three-dimensional smoothing unit 0201a and the second three-dimensional smoothing unit 0201b may be any order. The first three-dimensional smoothing unit 0201a and the second three-dimensional smoothing unit 0201b may be configured to set the first smoothing coefficient and the second smoothing coefficient first and thereafter perform the smoothing respectively.

As in the first embodiment, the weighted addition unit 0202 receives and sets a weight coefficient (step S1205). The weighted addition unit 0202 weights and adds up, for each voxel, the texture volume data and the smoothed volume data according to Equation (1) and obtains the combined volume data 0403 shown in Figure 4(c) (step S1206).

For example, even if the texture volume data 0401 shown in Figure 4(a) is insufficiently subjected to the smoothing and the smoothed volume data 0402 shown in Figure 4(b) is excessively subjected to the smoothing, by adding up both the volume data using the weighted addition unit 0202, it is possible to obtain the combined volume data 0403 subjected to smoothing intermediate between the texture volume data 0401 and the smoothed volume data 0402. By changing the weight coefficient, it is possible to obtain combined volume data having desired smoothing intensity intermediate between the smoothed volume data 0402 and the texture volume data 0401.

Thereafter, as in the first embodiment, the gradient calculating unit 0203 performs a gradient calculation for the combined volume data 0403 (step S1207). That is, the gradient calculating unit 0203 calculates, every time processed volume data is generated, luminance gradients of a respective generated plurality of pieces of the processed volume data. The rendering unit 0204 applies rendering processing to the combined volume data 0403 and generates a three-dimensional image (step S1208). That is, the rendering unit 0204 generates, every time an input of a weight coefficient is received via an interface, a three-dimensional image from the plurality of pieces of processed volume data. The generated three-dimensional image is changed to display data by the display data generating unit 0016 and displayed on the display device 0009.

Note that, in this embodiment, an effect of a weight coefficient set by the operator is basically the same as the effect in the first embodiment. In the texture volume data 0401 with low smoothing intensity, information such as wrinkles of fingers and a face of a fetus is retained but, on the other hand, unevenness remains on a boundary line of an image. In the smoothed volume data 0402 with high smoothing intensity, the boundary line of the image is smooth without unevenness but, on the other hand, information such as the wrinkles of the fingers and the face of the fetus is lost.

In this embodiment, as in the first embodiment, the ultrasonic diagnostic device includes an interface for adjusting a weight coefficient. The operator adjusts the weight coefficient and adjusts smoothing intensity of combined volume data by changing the weight coefficient in real time while looking at an image displayed on the display device 0009.

In this embodiment, as in the first embodiment, the ultrasonic diagnostic device may include an interface for changing a first smoothing coefficient and a second smoothing coefficient in real time. In this embodiment, the ultrasonic diagnostic device may include, for each coefficient, an interface same as the interface in the first embodiment or may be configured to receive a change of both the coefficients in one interface according to an instruction from the operator. Further, as in the first embodiment, the interface may be used as an interface for adjustment of a weight coefficient as well.

As explained above, according to this embodiment, in the three-dimensional image processing, combined volume data is generated by multiplying texture volume data and smoothed volume data, which are obtained by applying the smoothing to one tomographic volume data at smoothing intensities different from each other, with a weight coefficient set in advance and adding up the volume data. Then, the rendering processing is applied to the generated combined volume data to generate a three-dimensional image.

Therefore, as in the first embodiment, it is possible to easily obtain a three-dimensional image subjected to desired smoothing and obtain an optimum image according to necessity. Since it is possible to obtain an image having smoothing intensity intermediate between images smoothed at two different smoothing intensities, it is possible to set a smoothing level at a higher degree of freedom compared with the first embodiment. That is, the operator can have a wide span of adjustable range of smoothing for a three-dimensional image.

Otherwise, the second embodiment has a configuration same as the configuration of the first embodiment. Therefore, various effects same as the effects of the first embodiment are obtained. It is possible to obtain a three-dimensional image having a desired image quality at high speed without increasing a load on the device.

Note that, in the embodiment, the configuration including the two three-dimensional smoothing units 0201 is explained as an example. However, the configuration of the ultrasonic diagnostic device is not limited to this configuration. The ultrasonic diagnostic device may be configured to sequentially apply, with one three-dimensional smoothing unit 0201, smoothing to the same tomographic volume data using different smoothing coefficients and subject outputs of the smoothing to weighted addition.

As in the first embodiment, it is possible to three-dimensionally use an averaging filter, a low-pass filter, or a median filter as spatial smoothing in the three-dimensional smoothing units 0201a and 0201b. Expansion processing by dilation may be three-dimensionally performed.

«Third Embodiment»

Next, a third embodiment to which the present invention is applied is explained. In the second embodiment, the three-dimensional image processing unit 0014a subjects two kinds of volume data, which are obtained by applying the smoothing to an output of the three-dimensional coordinate conversion unit 0013 at different smoothing intensities, to the weighted addition and generates combined volume data. On the other hand, in this embodiment, a three-dimensional image processing unit adds up volume data after a plurality of kinds of filter processing, which are obtained by applying different kinds of filter processing to an output of the same three-dimensional coordinate conversion unit, using a predetermined weight coefficient and generates combined volume data.

The ultrasonic diagnostic device 0001 in this embodiment basically has a configuration same as the configuration of the ultrasonic diagnostic device 0001 in the first embodiment. However, since the three-dimensional image processing is different as explained above, the ultrasonic diagnostic device 0001 includes a three-dimensional image processing unit 0014b instead of the three-dimensional image processing unit 0014 in the first embodiment. In this embodiment, the three-dimensional image processing unit 0014b is mainly explained below. Note that, here, as an example, two kinds of processing, i.e., three-dimensional sharpening and three-dimensional smoothing are performed as different kinds of filter processing.

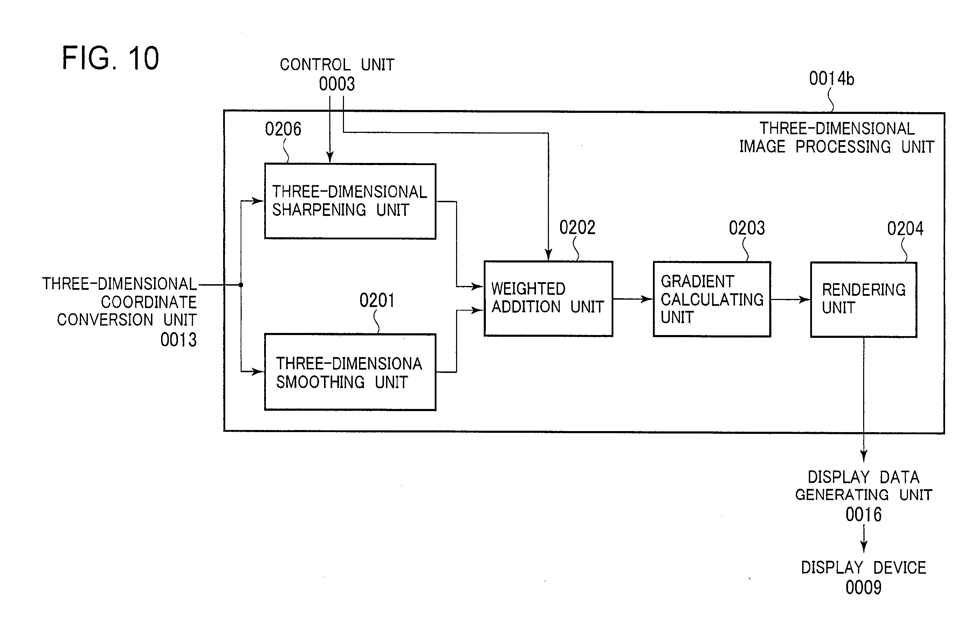

Figure 10 is a functional block diagram of the three-dimensional image processing unit 0014b in this embodiment. As shown in the figure, the three-dimensional image processing unit 2014 in this embodiment includes the three-dimensional smoothing unit 0201, a three-dimensional sharpening unit 0206, the weighted addition unit 0202, the gradient calculating unit 0203, and the rendering unit 0204.

The three-dimensional sharpening unit 0206 applies sharpening to tomographic volume data, which is output data of the three-dimensional coordinate conversion unit 0013. In this embodiment, volume data after the sharpening is referred to as texture volume data (sharpened volume data). As the sharpening performed here, there are sharpening in which a high-pass filter is three-dimensionally used, retraction processing by three-dimensional erosion processing, and the like. Spatially-sharpened volume data is generated by using the three-dimensional high-pass filter. Volume data with enhanced structure is generated by the retraction processing by the three-dimensional erosion processing.

The sharpening is performed using a sharpening coefficient for determining intensity of sharpening. Like the smoothing coefficient in the first embodiment, the sharpening coefficient is designated by an operator via the operation unit 0004 out of sharpening coefficients set in advance. The three-dimensional sharpening unit 0206 receives the designated sharpening coefficient via the control unit 0003 and sets the sharpening coefficient.

The three-dimensional smoothing unit 0201 is basically the same as the three-dimensional smoothing unit 0201 in the first embodiment. In this embodiment, volume data generated by applying the smoothing to tomographic volume data is referred to as smoothed volume data (smooth volume data). In this embodiment, as spatial smoothing, a low-pass filter, a median filter, or the like may be three-dimensionally used. Expansion processing by dilation may be three-dimensionally performed. In this way, in this embodiment, it is possible to give an arbitrary smoothing effect to a three-dimensional ultrasonic image.

A function of the weighted addition unit 0202 is basically the same as the function in the first embodiment. However, a plurality of pieces of volume data generated from the same tomographic volume data to be subjected to weighted addition are two volume data, i.e., the texture volume data subjected to the sharpening and the smoothed volume data subjected to the smoothing.

Functions of the other components (the gradient calculating unit 0203 and the rendering unit 0204) are basically the same as the functions in the first embodiment. As in the first and second embodiments, a configuration not including the gradient calculating unit 0203 is also possible.

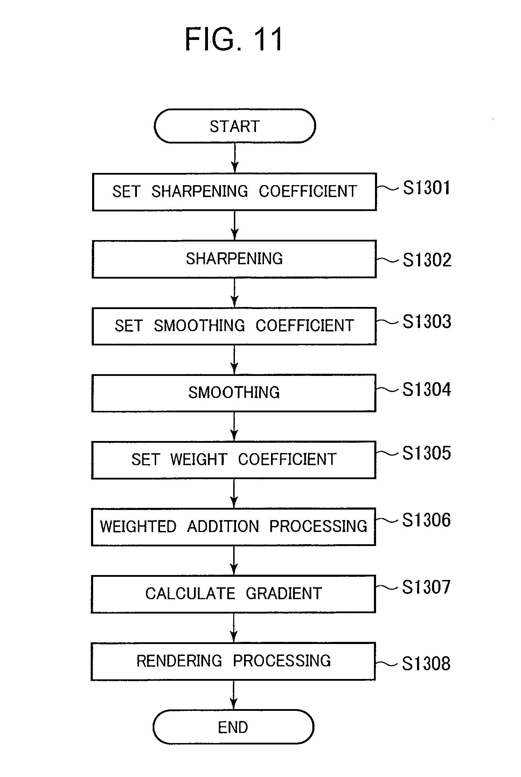

Next, a procedure of image processing by the three-dimensional image processing unit 0014b in this embodiment is explained using Figure 11. Figure 11 is a processing flow of the image processing by the three-dimensional image processing unit 0014b in this embodiment. As in the second embodiment, in order to explain an overview of an effect, volume data to be generated is explained using Figure 4.

The three-dimensional sharpening unit 0206 sets a sharpening coefficient (step S1301), applies the sharpening to tomographic volume data, which is output  data of the three-dimensional coordinate conversion unit 0013, and generates the texture volume data 0401 shown in Figure 4(a) (step S1302).

Next, the three-dimensional smoothing unit 0201 sets a second smoothing coefficient (step S1303), applies the smoothing to the tomographic volume data, which is the output data of the three-dimensional coordinate conversion unit 0013, and generates the smoothed volume data 0402 shown in Figure 4(b) (step S1304).

Note that processing order of the three-dimensional sharpening unit 0206 and the three-dimensional smoothing unit 0201 may be any order. The three-dimensional sharpening unit 0206 and the three-dimensional smoothing unit 0201 may be configured to set both the coefficients first and thereafter perform both the kinds of processing.

As in the first embodiment, the weighted addition unit 0202 receives and sets a weight coefficient (step S1305). Then, the weighted addition unit 0202 weights and adds up, for each voxel, the texture volume data and the smoothed volume data according to Equation (1) and obtains the combined volume data 0403 shown in Figure 4(c) (step S1306).

Thereafter, as in the first embodiment, the gradient calculating unit 0203 performs a gradient calculation for the combined volume data 0403 (step S1307). The rendering unit 0204 applies the rendering processing to the combined volume data 0403 and generates a three-dimensional image (step S1308). Note that the generated three-dimensional image is changed to display data by the display data generating unit 0016 and displayed on the display device 0009.

Note that, in this embodiment, the influence of a weight coefficient set by the operator is basically the same as the influence in the first embodiment. In this embodiment, as in the first embodiment, the ultrasonic diagnostic device includes an interface for adjusting a weight coefficient. The operator changes the weight coefficient in real time and adjusts the weight coefficient while looking at an image displayed on the display device 0009.

In this embodiment, as in the first embodiment, the ultrasonic diagnostic device may include an interface for changing, in real time, a smoothing coefficient used for the smoothing and a sharpening coefficient used for the sharpening.

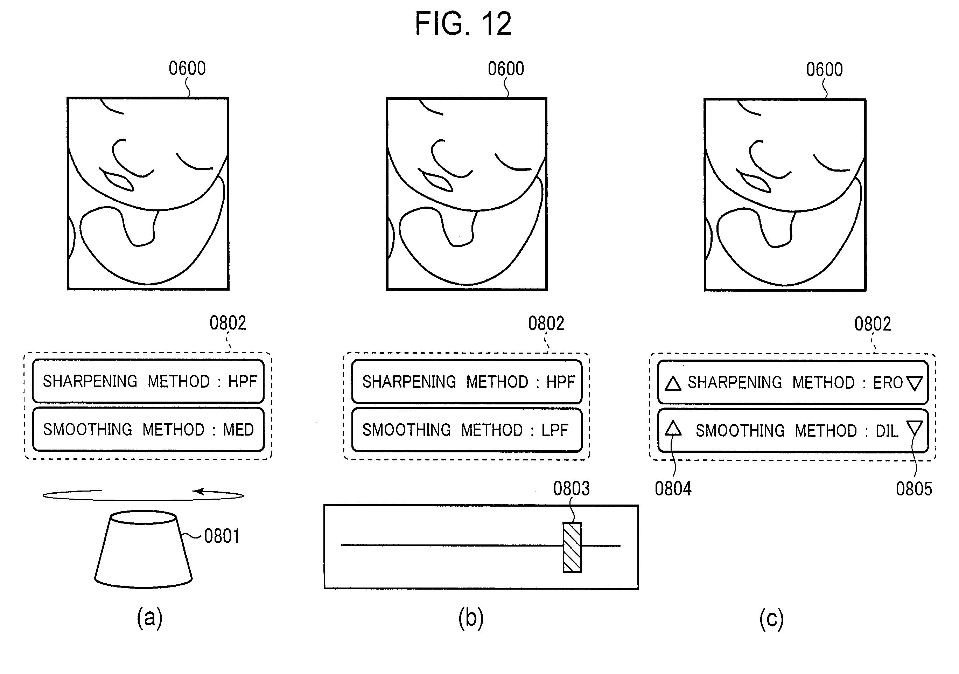

In this embodiment, the ultrasonic diagnostic device may include an interface with which the operator selects a processing method used for sharpening and a processing method used for smoothing. The interface with which the operator selects these processing methods is explained using Figure 12.

Figure 12(a) is an example in which the interface for selecting processing methods is configured by a changing dial 0801 included in the operation unit 0004, a processing method display region 0802, and the three-dimensional image display region 0600. The operation unit 0004 includes the changing dial 0801. The operator selects desired processing methods out of sharpening methods, smoothing methods, and the like prepared in advance by operating the changing dial 0801.

The display device 0009 includes the processing method display region 0802 and the three-dimensional image display region 0600. Note that the three-dimensional image display region 0600 is a region in which a three-dimensional image generated by the three-dimensional image processing unit 0014b is displayed.

The control unit 0003 displays, every time the operator sets or changes three-dimensional filter processing methods via the changing dial 0801, each of  the selected processing methods in the processing method display region 0802. The control unit 0003 notifies, every time the operator selects or changes processing methods via the changing dial 0801, the three-dimensional image processing unit 0014b of the processing methods. The three-dimensional image processing unit 0014b receives the notification and executes the changed processing and the weighted addition processing and the subsequent processing. The generated three-dimensional image is displayed in the three-dimensional image display region 0600. The operator sets optimum processing methods while checking the three-dimensional image displayed in the three-dimensional image display region 0600.

Figure 12(b) is an example in which the interface for selecting processing methods is configured by the touch panel on the display device 0009, the processing method display region 0802, and the three-dimensional image display region 0600. The display device 0009 includes a slide bar 0803 for receiving selection of processing methods. The operator selects desired processing methods out of the sharpening methods, the smoothing methods, and the like prepared in advance by operating the slide bar 0803 using a finger or the input device such as the mouse, the trackball, or the keyboard included in the operation unit 0004. Processing  performed by the control unit 0003 when the processing methods are selected by the operator is the same as the example shown in Figure 12(a).

Figure 12(c) is an example in which the interface for selecting processing methods is configured by the touch panel on the display device 0009, the processing method display region 0802, and the three-dimensional image display region 0600. The display device 0009 includes instruction regions 0804 and 0805 for receiving instructions for changing processing methods. The operator instructs a change of a processing method via the instruction region 0804 or 0805. The operator performs the instruction using a finger or the input device such as the mouse or the keyboard included in the operation unit 0004. Processing performed by the control unit 0003 when processing methods are selected or changed by the operator is the same as the example shown in Figure 12(a).

As explained above, according to this embodiment, in the three-dimensional image processing, combined volume data is generated by multiplying processed volume data, which are obtained by applying kinds of filter processing different from each other to one tomographic volume data, with a weight coefficient set in advance and adding up the volume data. Then, the rendering processing is applied to the generated combined volume data to generate a three-dimensional image.

Therefore, as in the first embodiment, the operator can arbitrarily change, by arbitrarily setting a weight coefficient, information concerning unevenness that the operator desires to suppress and fine form information that the operator desires to retain. The weight coefficient used during addition can be arbitrarily changed in real time. Therefore, it is possible to easily give a desired filter effect. Consequently, it is possible to easily obtain a three-dimensional image subjected to desired filter processing and obtain an optimum image according to necessity.

Otherwise, the third embodiment has a configuration same as the configuration of the first embodiment. Therefore, various effects same as the effects of the first embodiment are obtained. It is possible to obtain a three-dimensional image having a desired image quality at high speed without increasing a load on the device.

Note that, in the example explained in the embodiment, the sharpening and the smoothing are performed as the three-dimensional filter processing. However, the three-dimensional filter processing is not limited to this. Kinds of three-dimensional filter processing to be combined are not limited to two. The combination only has to be a combination of kinds of three-dimensional filter processing having different effects. Filter processing may be any kind and the number of kinds of filter processing may be any number.

When there are three or more kinds of three-dimensional filter processing to be executed, the weighted addition unit performs addition processing such that a total of weight coefficients respectively given to the kinds of three-dimensional filter processing is 1.

«Fourth Embodiment»

Next, a fourth embodiment to which the present invention is applied is explained. In the respective embodiments explained above, a plurality of pieces of volume data generated by applying the kinds of filter processing having different effects to the same tomographic volume data are subjected to the weighted addition using one weight coefficient to generate combined volume data. On the other hand, in this embodiment, a weight coefficient is changed according to positions of respective voxels to perform the weighted addition.

The ultrasonic diagnostic device 0001 in this embodiment basically has a configuration same as the configuration of the ultrasonic diagnostic device 0001 in the first embodiment. However, since three-dimensional image processing is different as explained above, the ultrasonic diagnostic device 0001 includes a three-dimensional image processing unit 0014c instead of the three-dimensional image processing unit 0014 in the first embodiment. In this embodiment, the three-dimensional image processing unit 0014c is mainly explained below. Note that filter processing having different effects may be any kinds. However, here, as an example, as in the second embodiment, the ultrasonic diagnostic device 0001 includes two three-dimensional smoothing units 0201. The three-dimensional smoothing units 0201 respectively perform smoothing at different smoothing intensities.

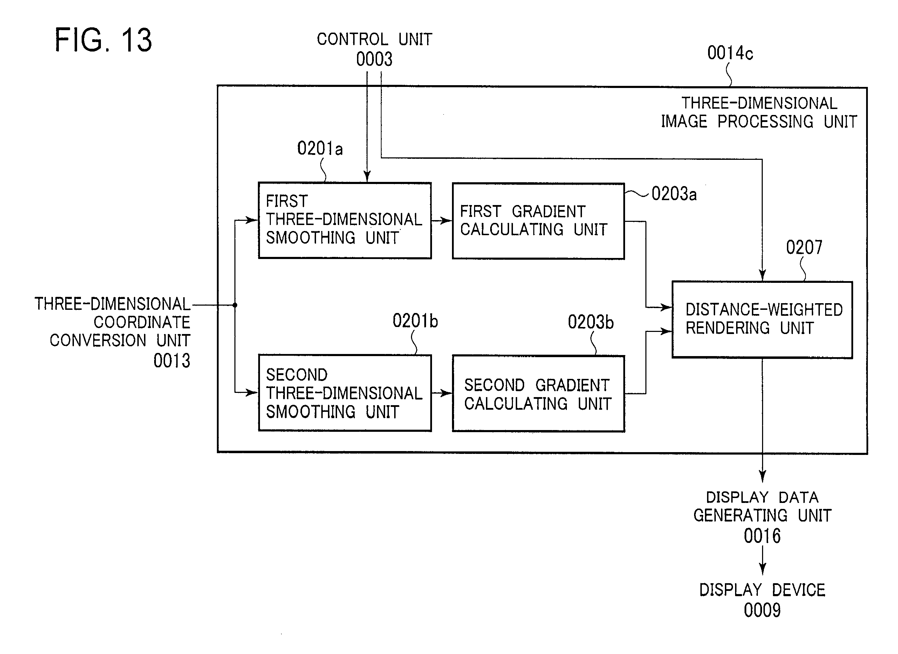

Figure 13 is a functional block diagram of the three-dimensional image processing unit 0014c in this embodiment. As shown in the figure, the three-dimensional image processing unit 0014c in this embodiment includes two three-dimensional smoothing units 0201 (0201a and 0201b), two gradient calculating units 0203 (0203a and 0203b), and a distance-weighted rendering unit (a rendering unit) 0207.

Each of functions of the two three-dimensional smoothing units 0201 (0201a and 0201b) is basically the same as the function in the second embodiment. As in the second embodiment, as respective smoothing coefficients used for smoothing, smoothing coefficients input by an operator via the operation unit 0004 are set by the control unit 0003.

Each of functions of the two gradient calculating units 0203 (0203a and 0203b) is basically the same as the function of the gradient calculating unit 0203 in the first embodiment. However, in this embodiment, the two gradient calculating units 0203 respectively generate gradient information of texture volume data, which is an output of the first three-dimensional smoothing unit 0201a, and smoothed volume data, which is an output of the second three-dimensional smoothing unit 0201b, rather than combined volume data. Here, for explanation, a side where the texture volume data is processed is referred to as first gradient calculating unit 0203a and a side where the smoothed volume data is processed is referred to as second gradient calculating unit 0203b.

The distance-weighted rendering unit (the rendering unit) 0207 performs volume rendering processing while subjecting the texture volume data and the smoothed volume data after calculation of a luminance gradient to weighted addition for each voxel according to a distance from a projection surface and generates a three-dimensional image. That is, the distance-weighted rendering unit (the rendering unit) 0207 changes a weight coefficient for first processed volume data and second processed volume data according to a distance to a processing target voxel from the projection surface.

The weight coefficient used here is a distance weight coefficient set according to position information of the processing target voxel. The operator inputs the distance weight coefficient via the operation unit 0004. The control unit 0003 notifies the distance weight coefficient.

Note that the rendering processing is performed using above Equation (2) and Equation (3). However, luminance information (a luminance value of an ith voxel present on a line of sight) $C_i$ and gradient information (a gradient value of the ith voxel present on the line of sight) $S_i$ are calculated according to the following Equation (7) and Equation (8):

$$C_i = K1_i \cdot C1_i + K2_i \cdot C2_i \qquad (7)$$

$$S_i = K1_i \cdot S1_i + K2_i \cdot S2_i \qquad (8)$$

where, $K1_i + K2_i = 1$.

Here, $C1_i$ and $C2_i$ are respectively a voxel value of the texture volume data and a voxel value of the smoothed volume data. $K1_i$ and $K2_i$ are distance weight coefficients set according to the distance from the projection surface.

As in the first, second, and third embodiments, a configuration not including the gradient calculating units 0203 (0203a and 0203b) is also possible. For example, 1.0 is always given as the gradient value $S_i$ in this case.

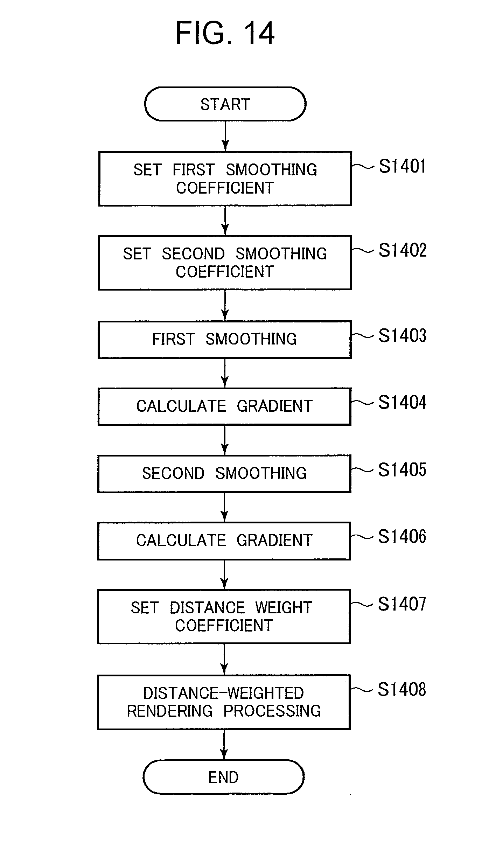

Next, a procedure of image processing in the characteristic three-dimensional image processing unit 0014c in this embodiment is explained using Figure 14. Figure 14 is a processing flow of the image processing of the three-dimensional image processing unit 0014c in this embodiment. As in the embodiments explained above, volume data to be generated is explained using Figure 4.

The first three-dimensional smoothing unit 0201a receives and sets a first smoothing coefficient (step S1401). The second three-dimensional smoothing unit 020b1 receives and sets a second smoothing coefficient (step S1402). As in the second embodiment, the first smoothing coefficient and the second smoothing coefficient are respectively designated by the operator via the operation unit 0004 out of smoothing coefficients set in advance and are notified by the control unit 0003. As explained above, as the second smoothing coefficient, a smoothing coefficient larger than the first smoothing coefficient is set.

Subsequently, the first three-dimensional smoothing unit 0201a applies the smoothing to tomographic volume data, which is output data of the three-dimensional coordinate conversion unit 0013, and generates the volume data (the texture volume data) 0401 subjected to the weak smoothing shown in Figure 4(a) (step S1403). The first gradient calculating unit 0203a calculates a luminance gradient of the texture volume data (step S1404).

The second three-dimensional smoothing unit 0201b applies the smoothing to the tomographic volume data and generates the smoothed volume data 0402 subjected to the intense smoothing shown in Figure 4(b) (step S1405). The second gradient calculating unit 0203b calculates a luminance gradient of the smoothed volume data (step S1406).

Note that, in the processing flow explained above, processing order of the setting of the respective smoothing coefficient, the respective kinds of smoothing, and the respective gradient calculations may be any order as long as order for setting the smoothing coefficients, performing the smoothing, and performing the gradient calculations is observed.

The three-dimensional image processing unit 0014 receives and sets a distance weight coefficient (step S1407). The distance-weighted rendering unit 0207 performs volume rendering processing while subjecting the texture volume data and the smoothed volume data to weighted addition for each voxel using the set distance weight coefficient and according to Equations (2), Equation (3), Equation (7), and Equation (8) and generates a three-dimensional image. The generated three-dimensional image is changed to display data by the display data generating unit 0016 and displayed on the display device 0009.

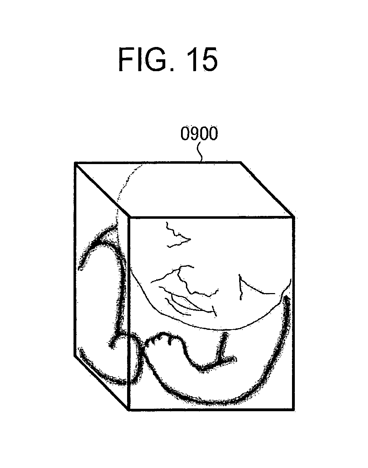

As explained above, in this embodiment, the weight coefficient can be changed according to the distance. For example, for a voxel at a close distance from the projection surface, $K1_i$ is set large and weight of the texture volume data having low smoothing intensity is set large. For a voxel at a far distance from the projection surface, $K2_i$ is set large and weight of the smoothed volume data having high smoothing intensity is set large. A conceptual diagram of volume data 0900 generated during processing in this case is shown in Figure 15.

As shown in the figure, a degree of smoothing is moderately suppressed only in a region of interest in a depth direction. Regions other than the region of interest are excessively smoothed. In this way, it is possible to build a three-dimensional image by refining the volume data at the near distance and blurring the volume data at the far distance.

Note that, here, as an example of the three-dimensional filter processing for the tomographic volume data, the smoothing performed at different smoothing intensities are explained. However, the three-dimensional filter processing is not limited to this smoothing. As in the third embodiment, different kinds of three-dimensional filter processing may be applied. Kinds of three-dimensional filter processing having different effects to be applied are not limited to two kinds.

As explained above, according to this embodiment, in addition to the effects of the respective embodiments explained above, it is possible to change a degree of smoothing according to the distance. Therefore, by excessively smoothing voxel data in regions other than a region of interest that the operator desires to look at, it is possible to obtain a three-dimensional image in which the region of interest is more easily recognized. That is, it is possible to improve visibility of the region of interest in the depth direction.

All the kinds of processing in the respective embodiments explained above are not limitedly applied to a tomographic image and can be applied to a three-dimensional blood flow, elasticity information, and luminance information enhanced by an ultrasound contrast agent that can be acquired by the ultrasonic diagnostic device.

In the respective embodiments explained above, the ultrasonic diagnostic device 0001 does not have to include the functions of the respective units (the tomographic information calculating unit 0011, the three-dimensional coordinate conversion unit 0013, the three-dimensional image processing unit 0014, the arbitrary tomographic image creating unit 0015, and the display data generating unit 0016) for generating tomographic volume data from RF signal frame data and further generating a two-dimensional image and a three-dimensional image. For example, the functions may be built on an information processing device capable of transmitting and receiving data to and from the ultrasonic diagnostic device 0001 and independent from the ultrasonic diagnostic device 0001. A part of the respective functions may be built on the independent information processing device.

Further, in the respective embodiments explained above, the ultrasound diagnostic device is explained as an example of a test device to which the present invention is applied. However, the present invention is not limited to the ultrasonic diagnostic device. The present invention can be applied to various test devices that generate a three-dimensional image from tomographic volume data such as an MRI device and an X-ray CT device.

Reference Signs List

0001    Ultrasonic diagnostic device

0002    Ultrasonic probe

0003    Control unit

0004    Operation unit

0005    Transmitting unit

0006    Receiving unit

0007    Transmission and reception control unit

0008    Phasing addition unit

0009    Display device (display unit)

0010    Object

0011    Tomographic information calculating unit

0012    Three-dimensional tomographic data storing unit

0013    Three-dimensional coordinate conversion unit

0014    Three-dimensional image processing unit

0014a    Three-dimensional image processing unit

| | |
|---|---|
| 0014b | Three-dimensional image processing unit |
| 0014c | Three-dimensional image processing unit |
| 0015 | Arbitrary tomographic image creating unit |
| 0016 | Display data generating unit |
| 0201 | Three-dimensional smoothing unit |
| 0201a | First three-dimensional smoothing unit |
| 0201b | Second three-dimensional smoothing unit |
| 0202 | Weighted addition unit |
| 0203 | Gradient calculating unit |
| 0203a | First gradient calculating unit |
| 0203b | Second gradient calculating unit |
| 0204 | Rendering unit |
| 0206 | Three-dimensional sharpening unit |
| 0207 | Distance-weighted rendering unit |
| 0401 | Texture volume data |
| 0402 | Smoothed volume data |
| 0403 | Combined volume data |
| 0501 | Three-dimensional image |
| 0502 | Three-dimensional image |
| 0503 | Three-dimensional image |
| 0600 | Three-dimensional image display region |
| 0601 | Changing dial |
| 0602 | Coefficient display region |
| 0603 | Slide bar |
| 0604 | Increase instruction region |
| 0605 | Reduction instruction region |
| 0701 | Changing dial |
| 0702 | Coefficient display region |
| 0703 | Slide bar |

0704    Increase instruction region

0705    Reduction instruction region

0801    Changing dial

0802    Processing method display region

0803    Slide bar

0804    Instruction region

0805    Instruction region

**Claims**

1.  An ultrasonic diagnostic device comprising:

    an ultrasonic probe;
    a transmitting unit configured to transmit a driving signal to the ultrasonic probe;
    a three-dimensional tomographic data generating unit configured to generate, using an ultrasonic signal from a test target measured by the ultrasonic probe, tomographic volume data of the test target;
    a three-dimensional image processing unit configured to generate a three-dimensional image from the tomographic volume data; and
    a display unit configured to display the three-dimensional image generated by the three-dimensional image processing unit, wherein
    the three-dimensional image processing unit includes:

       a smoothing unit configured to apply spatial smoothing to the tomographic volume data and generate smoothed volume data;
       a weighted addition unit configured to multiply, when the smoothed volume data is generated, the tomographic volume data and the smoothed volume data with a weight coefficient and add up the volume data to generate combined volume data; and
       a rendering unit configured to apply, when the combined volume data is generated, rendering processing to the combined volume data and generate the three-dimensional image.

2.  An ultrasonic diagnostic device comprising:

    an ultrasonic probe;
    a transmitting unit configured to transmit a driving signal to the ultrasonic probe;
    a three-dimensional tomographic data generating unit configured to generate, using an ultrasonic signal from a test target measured by the ultrasonic probe, tomographic volume data of the test target;
    a three-dimensional image processing unit configured to generate a three-dimensional image from the tomographic volume data; and
    a display unit configured to display the three-dimensional image generated by the three-dimensional image processing unit, wherein
    the three-dimensional image processing unit includes:

       a filter processing unit configured to apply each of a plurality of kinds of three-dimensional filter processing to the tomographic volume data and generate processed volume data by the respective three-dimensional filter processing;
       a weighted addition unit configured to multiply, when a plurality of pieces of the processed volume data are generated, the plurality of pieces of processed volume data with a weight coefficient and add up the plurality of pieces of processed volume data to generate combined volume data; and
       a rendering unit configured to apply, when the combined volume data is generated, rendering processing to the combined volume data and generate the three-dimensional image.

3. The ultrasonic diagnostic device according to claim 1 or 2, further comprising an interface for receiving an input of the weight coefficient from an operator, wherein
the weighted addition unit generates the combined volume data when the input of the weight coefficient is received via the interface.

4. The ultrasonic diagnostic device according to claim 1 or 2, further comprising a gradient calculating unit configured to calculate, when the combined volume data is generated, a luminance gradient of the combined volume data, wherein
the rendering unit executes the rendering processing using the luminance gradient calculated by the gradient calculating unit.

5. An ultrasonic diagnostic device comprising:

an ultrasonic probe;
a transmitting unit configured to transmit a driving signal to the ultrasonic probe;
a three-dimensional tomographic data generating unit configured to generate, using an ultrasonic signal from a test target measured by the ultrasonic probe, tomographic volume data of the test target;
a three-dimensional image processing unit configured to generate a three-dimensional image from the tomographic volume data; and
a display unit configured to display the three-dimensional image generated by the three-dimensional image processing unit, wherein

the three-dimensional image processing unit includes:

a filter processing unit configured to apply each of a plurality of kinds of three-dimensional filter processing to the tomographic image and generate processed volume data in the respective kinds of three-dimensional filter processing; and
a rendering unit configured to apply, when the processed volume data is generated, rendering processing to a plurality of pieces of the generated processed volume data while multiplying the processed volume data with a weight coefficient and adding up the processed volume data for each constituent voxel to generate the three-dimensional image.

6. The ultrasonic diagnostic device according to claim 5, wherein the rendering unit changes the weight coefficient for first processed volume data and second processed volume data among the plurality of pieces of processed volume data according to a distance from a projection surface to a processing target voxel.

7. The ultrasonic diagnostic device according to claim 5, further comprising an interface for receiving an input of the weight coefficient from an operator, wherein
the rendering unit generates the three-dimensional image from the plurality of pieces of processed volume data when the input of the weight coefficient is received via the interface.

8. The ultrasonic diagnostic device according to claim 5, further comprising a gradient calculating unit configured to calculate, when the processed volume data is generated, luminance gradients of the respective generated plurality of pieces of processed volume data, wherein
the rendering unit applies the rendering processing to the respective processed volume data using the respective luminance gradients calculated by the gradient calculating unit.

9. The ultrasonic diagnostic device according to claim 2 or 5, wherein the plurality of kinds of three-dimensional filter processing include first smoothing in which a first smoothing coefficient is used and second smoothing in which a second smoothing coefficient different from the first smoothing coefficient is used.

10. The ultrasonic diagnostic device according to claim 2 or 5, wherein the plurality of kinds of three-dimensional filter processing includes spatial smoothing and sharpening.

11. The ultrasonic diagnostic device according to claim 10, wherein the spatial smoothing is expansion processing by dilation.

12. The ultrasonic diagnostic device according to claim 10, further comprising an interface for receiving an input of a processing method used for the smoothing and a processing method used for the sharpening from an operator, wherein

in the smoothing, when the input of the processing method is received, smoothed volume data is generated by the processing method, and

in the sharpening, when the input of the processing method is received, sharpened volume data is generated by the processing method.

**13.** The ultrasonic diagnostic device according to claim 1, further comprising an interface for receiving an input of a smoothing coefficient used for the smoothing from an operator, wherein

in the smoothing, when the input of the smoothing coefficient is received, the smoothed volume data is generated using the smoothing coefficient.

**14.** The ultrasonic diagnostic device according to claim 2 or 5, further comprising an interface for receiving an input of a filter coefficient used for the three-dimensional filter processing from an operator, wherein

the filter processing unit generates, when the input of the filter coefficient is received, the processed volume data using the filter coefficient.

**15.** An image processing method in an ultrasonic diagnostic device for transmitting ultrasound to a test target, applying image processing to tomographic volume data of the test target generated using a received ultrasonic signal, and generating a three-dimensional image, the image processing method comprising:

applying spatial smoothing to the tomographic volume data and generating smoothed volume data;
multiplying the tomographic volume data and the smoothed volume data with a weight coefficient and adding up the volume data to generate combined volume data; and
applying rendering processing to the combined volume data and generating the three-dimensional image.

**FIG. 1**

0001

ULTRASONIC DIAGNOSTIC DEVICE

```
               ┌─0005            ┌─0007              ┌─0004            ┌─0003
         ┌─────────────┐  ┌──────────────────┐  ┌──────────────┐  ┌──────────────┐
         │ TRANSMITTING │◄─│ TRANSMISSION AND │  │ OPERATION    │─►│ CONTROL UNIT │
         │ UNIT         │  │ RECEPTION CONTROL│  │ UNIT         │  │              │
         └─────────────┘  │ UNIT             │  └──────────────┘  └──────────────┘
                          └──────────────────┘
               ┌─0006            ┌─0008
         ┌─────────────┐  ┌──────────────────┐
         │ RECEIVING   │◄─│ PHASING ADDITION │
         │ UNIT        │─►│ UNIT             │
         └─────────────┘  └──────────────────┘

        ┌─0011            ┌─0012              ┌─0013            ┌─0014
  ┌──────────────┐  ┌──────────────────┐  ┌──────────────┐  ┌──────────────────┐
  │ TOMOGRAPHIC  │  │ THREE-DIMENSIONAL│  │ THREE-       │  │ THREE-DIMENSIONAL │
  │ INFORMATION  │─►│ TOMOGRAPHIC DATA │─►│ DIMENSIONAL  │─►│ IMAGE PROCESSING  │
  │ CALCULATING  │  │ STORING UNIT     │  │ COORDINATE   │  │ UNIT              │
  │ UNIT         │  │                  │  │ CONVERSION   │  │                   │
  └──────────────┘  └──────────────────┘  │ UNIT         │  └──────────────────┘
                                          └──────────────┘
                                   ┌─0015            ┌─0016
                             ┌──────────────┐  ┌──────────────┐
                             │ ARBITRARY    │  │ DISPLAY-DATA │
                             │ TOMOGRAPHIC  │─►│ GENERATING   │
                             │ IMAGE        │  │ UNIT         │
                             │ CREATING UNIT│  └──────────────┘
                             └──────────────┘
```

┌─0002
│ PROBE
┌─0010
│ OBJECT

┌─0009
│ DISPLAY DEVICE

EP 2 668 905 A1

**FIG. 2**

CONTROL UNIT
0003

THREE-DIMENSIONAL
IMAGE PROCESSING UNIT
0014

0202

0203

0204

THREE-DIMENSIONAL
COORDINATE
CONVERSION UNIT
0013

0201

WEIGHTED
ADDITION UNIT

GRADIENT
CALCULATING
UNIT

RENDERING
UNIT

THREE-DIMENSIONAL
SMOOTHING UNIT

DISPLAY DATA
GENERATING UNIT
0016

DISPLAY DEVICE
0009

# FIG. 3

START

SET SMOOTHING COEFFICIENT ⎯ S1101

SMOOTHING ⎯ S1102

SET WEIGHT COEFFICIENT ⎯ S1103

WEIGHED ADDITION PROCESSING ⎯ S1104

CALCULATE GRADIENT ⎯ S1105

RENDERING PROCESSING ⎯ S1106

END

# FIG. 4

0401

0402

0403

(a)

(b)

(c)

# FIG. 5

(a)　　　　　　(b)　　　　　　(c)

EP 2 668 905 A1

## FIG. 6

0600

0602

WEIGHT COEFFICIENT : 0.2

0601

(a)

0600

0602

WEIGHT COEFFICIENT : 0.2

0603

(b)

0600

0602

△ WEIGHT COEFFICIENT : 0.2 ▽

0604                                    0605

(c)

EP 2 668 905 A1

FIG. 7

0600

0600

0600

0702

0702

0702

SMOOTHING COEFFICIENT : 3

SMOOTHING COEFFICIENT : 3

△ SMOOTHING COEFFICIENT : 3 ▽

0704

0705

0701

0703

(a)

(b)

(c)

# FIG. 8

CONTROL UNIT
0003

0014a

THREE-DIMENSIONAL
IMAGE PROCESSING UNIT

0201a

FIRST THREE-
DIMENSIONAL
SMOOTHING UNIT

THREE-DIMENSIONAL
COORDINATE
CONVERSION UNIT
0013

0201b

SECOND THREE-
DIMENSIONAL
SMOOTHING UNIT

0202

WEIGHTED
ADDITION UNIT

0203

GRADIENT
CALCULATING
UNIT

0204

RENDERING
UNIT

DISPLAY DATA
GENERATING UNIT
0016

DISPLAY DEVICE
0009

EP 2 668 905 A1

# FIG. 9

START

SET FIRST SMOOTHING COEFFICIENT — S1201

SMOOTHING — S1202

SET SECOND SMOOTHING COEFFICIENT — S1203

SMOOTHING — S1204

SET WEIGHT COEFFICIENT — S1205

WEIGHTED ADDITION PROCESSING — S1206

CALCULATE GRADIENT — S1207

RENDERING PROCESSING — S1208

END

FIG. 10

THREE-DIMENSIONAL IMAGE PROCESSING UNIT 0014b

CONTROL UNIT 0003

0206 — THREE-DIMENSIONAL SHARPENING UNIT

0201 — THREE-DIMENSIONAL SMOOTHING UNIT

THREE-DIMENSIONAL COORDINATE CONVERSION UNIT 0013

0202 — WEIGHTED ADDITION UNIT

0203 — GRADIENT CALCULATING UNIT

0204 — RENDERING UNIT

DISPLAY DATA GENERATING UNIT 0016

DISPLAY DEVICE 0009

# FIG. 11

START

SET SHARPENING COEFFICIENT — S1301

SHARPENING — S1302

SET SMOOTHING COEFFICIENT — S1303

SMOOTHING — S1304

SET WEIGHT COEFFICIENT — S1305

WEIGHTED ADDITION PROCESSING — S1306

CALCULATE GRADIENT — S1307

RENDERING PROCESSING — S1308

END

FIG. 12

# FIG. 13

THREE-DIMENSIONAL
IMAGE PROCESSING UNIT
0014c

CONTROL UNIT
0003

0201a — FIRST THREE-DIMENSIONAL SMOOTHING UNIT

0203a — FIRST GRADIENT CALCULATING UNIT

THREE-DIMENSIONAL COORDINATE CONVERSION UNIT 0013

0207 — DISTANCE-WEIGHTED RENDERING UNIT

0201b — SECOND THREE-DIMENSIONAL SMOOTHING UNIT

0203b — SECOND GRADIENT CALCULATING UNIT

DISPLAY DATA GENERATING UNIT 0016

DISPLAY DEVICE 0009

EP 2 668 905 A1

# FIG. 14

START

↓

SET FIRST SMOOTHING COEFFICIENT — S1401

↓

SET SECOND SMOOTHING COEFFICIENT — S1402

↓

FIRST SMOOTHING — S1403

↓

CALCULATE GRADIENT — S1404

↓

SECOND SMOOTHING — S1405

↓

CALCULATE GRADIENT — S1406

↓

SET DISTANCE WEIGHT COEFFICIENT — S1407

↓

DISTANCE-WEIGHTED RENDERING PROCESSING — S1408

↓

END

# FIG. 15

0900

**INTERNATIONAL SEARCH REPORT**

| International application No. |
|---|
| PCT/JP2012/000314 |

A. CLASSIFICATION OF SUBJECT MATTER
*A61B8/00*(2006.01)i

According to International Patent Classification (IPC) or to both national classification and IPC

B. FIELDS SEARCHED

Minimum documentation searched (classification system followed by classification symbols)
A61B8/00

Documentation searched other than minimum documentation to the extent that such documents are included in the fields searched
Jitsuyo Shinan Koho          1922–1996   Jitsuyo Shinan Toroku Koho   1996–2012
Kokai Jitsuyo Shinan Koho    1971–2012   Toroku Jitsuyo Shinan Koho   1994–2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X/A | JP 2006-51202 A (Toshiba Corp.), 23 February 2006 (23.02.2006), (Family: none) | 1,15/2-14 |
| A | JP 2004-129773 A (Hitachi Medical Corp.), 30 April 2004 (30.04.2004), & US 2004/0073112 A1 | 1-15 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 26 March, 2012 (26.03.12) | 03 April, 2012 (03.04.12) |

| Name and mailing address of the ISA/ Japanese Patent Office | Authorized officer |
|---|---|
| Facsimile No. | Telephone No. |

Form PCT/ISA/210 (second sheet) (July 2009)

**EP 2 668 905 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007229283 A
- JP 2009061182 A
- JP 2006130071 A